# EUROPEAN PATENT APPLICATION

(11) **EP 4 786 584 A1**
(43) Date of publication of application: **05.08.2026**
(21) Application number: 24870792.9
(22) Date of filing: 25.09.2024
(51) Int. Cl.: C12N 9/12, C12N 9/00, C12N 15/54, C12Q 1/6869

(54) **NUCLEIC ACID POLYMERASE FOR SINGLE-MOLECULE SEQUENCING AND MUTANT THEREOF**

(30) Priority: 25.09.2023 CN 202311247387
(71) Applicant: Geneus Technologies (Chengdu) Co., Ltd., Chengdu, Sichuan 610041 (CN)
(72) Inventor: ZOU, Yaozhong, Chengdu, Sichuan 610041 (CN); LUO, Ling, Chengdu, Sichuan 610041 (CN); CHEN, Que, Chengdu, Sichuan 610041 (CN); QIN, Ronghuo, Chengdu, Sichuan 610041 (CN); MA, Bo, Chengdu, Sichuan 610041 (CN); LUO, Jie, Chengdu, Sichuan 610041 (CN); LIU, Yang, Chengdu, Sichuan 610041 (CN); WANG, Shan, Chengdu, Sichuan 610041 (CN); DENG, Xu, Chengdu, Sichuan 610041 (CN); LONG, Shuiying, Chengdu, Sichuan 610041 (CN); WANG, Yan, Chengdu, Sichuan 610041 (CN); CHEN, Dong, Chengdu, Sichuan 610041 (CN)
(74) Representative: Di Giovine, Paolo
(86) International application number: PCT/CN2024/121135
(87) International publication number: WO 2025/067248

(57) **Abstract**

Provided is a novel nucleic acid polymerase that can be used for single-molecule sequencing technology. The foregoing polymerase belongs to the Phi29 polymerase family, but the sequence homology thereof with other reported polymerases for sequencing technology is less than 60%. After characterization and identification, the polymerase shows good comprehensive performance in aspects such as thermal stability, extension capability at room temperature, extension processivity, ability to utilize modified nucleotide substrates/chain replacement function catalytic kinetics for same, and electrolyte resistance, and is suitable for single-molecule sequencing technology based on the "sequencing by synthesis" scheme. A beneficial mutant strain of the wild-type polymerase is further provided, which exhibits better kinetic characteristics and can increase sequencing accuracy and throughput.

## Description

### Cross-reference of Related Application

This application claims priority of the Chinese patent application no. 202311247387.6 filed on September 25, 2023, which is hereby incorporated herein by reference in its entirety.

### Technical Field

The present invention belongs to the field of single-molecule sequencing based on a "sequencing-by-synthesis" scheme and engineering of a nucleic acid polymerase required therefor. Specifically, the present invention characterizes and identifies a novel polymerase suitable for the single-molecule sequencing technology, and through optimization and modification by enzyme engineering, obtains a polymerase mutant with improved performance in various aspects.

### Background

In recent years, the single-molecule sequencing technology is emerging and shows great application prospects. The so-called single-molecule sequencing refers to that, from the perspective of signal acquisition, a sufficiently identifiable sequencing signal from a nucleic acid base can be obtained based on only one nucleic acid molecule and without amplification to achieve the purpose of sequencing. Compared with traditional sequencing technologies based on multiple copies of a nucleic acid molecule, the single-molecule sequencing shows significant advantages in various aspects: 1. Amplification is not needed for signal detection, which avoids the "amplification preference" problem of the traditional sequencing technologies in the process of increasing the number of copies of a nucleic acid molecule to be tested. Thus, the detection for a complex sequencing sample has a better coverage rate with better uniformity and completeness. It is also conducive to a better detection sensitivity for a low-abundance sample. 2. The single-molecule sequencing requires only a single nucleic acid molecule to give a sequence signal, which avoids the problem of the traditional sequencing technologies that as signals from multiple molecules gradually become desynchronized, the sequencing process is forced to be promptly stopped, thereby greatly increasing a sequence length that can be obtained for each sequencing. The leap in read length has significant advantages for subsequent sequence assembly and acquisition of information on structural variation of a gene, thereby allowing for near-perfect interpretation of a complex genome and of a metagenome, and making it possible for the sequencing technology to be used in clinical applications on a large scale. 3. The principle of the single-molecule sequencing technology supports simultaneous conduction of a biochemical reaction and signal acquisition during a sequencing process, without pause. This "real-time" feature makes the sequencing process more convenient and efficient. In addition, the single-molecule sequencing can be combined with nanopore-based detection that does not require fluorescent labeling, laying a solid foundation for a significant reduction in sequencing costs.

The single-molecule sequencing, based on its significant advantages/potential in terms of data quality, detection cost, convenience, and timeliness, represents the future technological development direction in the field of sequencing, and is of great significance for the sequencing technology to be used in clinical applications on a large scale, as well as for advancing the development of precision medicine and promoting a surge in related markets. In the current sequencing field, the single-molecule sequencing technology is mainly based on two schemes. One scheme is based on the direct use of a nanopore to read a base in a nucleic acid chain, which is represented by the one adopted by Oxford Nanopore, United Kingdom. The other scheme is based on the way of "sequencing-by-synthesis", which achieves the purpose of sequencing by monitoring the type of nucleotide used in each step of a synthesis reaction during replication of a nucleic acid chain with a nucleic acid polymerase; this scheme is represented by the ones adopted by Pacific Biosciences, United States (based on a fluorescence signal) and the sequencing department of Roche (based on a current signal from a nanopore). Compared with the scheme of directly reading a nucleic acid base, the single-molecule sequencing technology with "sequencing-by-synthesis" is based on traditional, mature biochemical means and generates a simpler and clearer sequencing signal, in which the number of base corresponds directly to the number of signal (that is, each base corresponds to a signal segment with clear start and end boundaries), which is more conducive to the detection of a base homopolymer in a complex genome. Thus, the final sequencing accuracy is high and the high requirements of clinical applications are better met. With the increasing maturity of the technical scheme, the industry generally expects that the single-molecule "sequencing-by-synthesis" technology will see a surge in the clinical application market in the next few years, and promote the entire medical industry to enter a new era of precision medicine while realizing huge market benefits.

For the "sequencing-by-synthesis" scheme, a nucleic acid polymerase drives the entire biochemical process in the sequencing, with its importance self-evident. To implement this scheme based on a single molecule, higher requirements on the comprehensive performance of a polymerase are needed. These requirements include: 1. The polymerase can achieve efficient extension with a modified nucleotide. To date, all single-molecule "sequencing-by-synthesis" schemes require a special modification to a nucleotide involved in synthesis to obtain a base signal. The efficient utilization of a modified nucleotide by a polymerase is a prerequisite for sequencing to achieve certain efficiency and obtain a sufficient read length. 2. The catalytic activity of the polymerase shows kinetic characteristics that match well with the signal detection means. For the real-time sequencing-by-synthesis technology, a sequence signal corresponds to a biochemical event after a polymerase captures a nucleotide and before the end of a chemical reaction for synthesis. If the kinetic parameters of an enzyme are less optimized and a synthesis reaction is completed too quickly, it is easy to result in that the sequence signal fail to be captured in time or is difficult to be distinguished from an interfering signal, leading to a sequencing error. If a synthesis reaction is completed too slowly, this will not only reduce the sequencing efficiency, but also easily leads to spontaneous dissociation of a nucleotide from a polymerase before the synthesis reaction is completed, generating an invalid signal in sequencing and reducing the sequencing accuracy. 3. Desired extension processivity, that is, the number of base that one polymerase molecule can continuously add for extension before releasing the template molecule. In a single-molecule sequencing scheme, sequence signals were obtained with one polymerase corresponding to one nucleic acid template molecule. Once the polymerase releases the template molecule, sequencing of the same template molecule cannot be restarted in a single-molecule setting, which significantly affects the read length and data output. 4. Excellent thermal stability. Likewise, in a sequencing system where one polymerase molecule corresponds to one nucleic acid template chain to be tested, the polymerase needs to have certain thermal stability, so that it can maintain its activity for a sufficient long time under the sequencing conditions, and would not be denatured and inactivated prematurely due to poor thermal stability; this is the basis for a sequencing platform to operate continuously to obtain a sufficient read length and sequencing data. 5. Excellent strand displacement function. Some polymerases have an unwinding function similar to helicase in addition to their activity of extending a DNA at the 3' end. In the process of extension, if the template region ahead is in a double-stranded form, such polymerases can remove the complementary strand from the template through unwinding, and replace it with a newly synthesized product strand complementary to the template strand to form a new double-stranded molecule. Such a strand displacement activity is not essential for sequencing-by-synthesis, but a polymerase possessing this activity has considerable advantages in sequencing. For example, a sample to be sequenced can be in a double-stranded form and does not need to be specially treated to form a single strand. As another example, a polymerase having the strand displacement function can carry out continuous rolling circle extension on a circular template, so as to achieve multiple rounds of sequencing for the same sequence to improve the sequencing accuracy. Therefore, a polymerase having the excellent strand displacement function is also often desired for a single-molecule sequencing-by-synthesis platform. 6. Other characteristics of the polymerase, including operating temperature and salt concentration compatible with the sequencing platform environment, excellent fidelity, and low level of exonuclease activity.

The Phi29 polymerase (or another polymerase with similar properties), based on its excellent processivity and strand displacement function, has become the enzyme of choice for a single-molecule sequencing platform. To further improve its performance in sequencing applications, the research and development teams, with Pacific Biosciences (PacBio), United States, as a representative example, have extensively engineered the wild-type Phi29 polymerase by introducing beneficial mutations at multiple sites of the enzyme, and obtained mutants with improved performance in various aspects over the wild-type polymerase, which have been used in its sequencing platform. The sequencing department of Roche has chosen a pol6 family polymerase and, through subsequent optimization and engineering, obtained a polymerase suitable for its sequencing platform. Despite these successful cases, different sequencing platforms generally have significantly different specific requirements on the performance of a polymerase.

### Summary of the Invention

In an aspect, provided herein is a DNA polymerase mutant comprising any one of the following mutations or any combination thereof relative to a wild-type polymerase having an amino acid sequence shown in SEQ ID NO: 1:
T272S, T272Y, A273K, T371D, Y372F, L265A, I379K, L398T, M377Y, E378Q, T384K, L398S, E493K, E493Q, K491R, Q389R, S397T, L468I, G512Q, Y161L, M198A, L318N, Q313C, S319K, E162L, S24R, I67A, M114T, T210D, E233K, K491R, K116R, K315R, K316R, S319C, N317K, S319R, S319F, E493K, L265A, E378Q, L468I, L265A, N263Q, E493K, D516K, N151C, T384S, L265A, T384N, Q193C, I502L, D516A, Q193S, N151V, D516Y, D516G, D516N, N151L, Q193L, E509V, D516T, H518L, N151Q, D516S, N151C, T243S, T243V, D516K, N151C, T384S, T384N, Q193C, I502L, L265A, D516A, Q193S, N151V, D516Y, D516G, D516N, N151L, Q193L, E509V, D516T, H518L, E493K, N151Q, D516S, N151C, T243S, K491R, and T243V.

In some embodiments, the DNA polymerase mutant comprises any one of the following mutations or any combination thereof relative to the wild-type polymerase, thereby increasing its extension rate with a modified nucleotide: T272S, T272Y, A273K, T371D, Y372F, L265A, I379K, L398T, M377Y, E378Q, T384K, L398S, E493K, E493Q, K491R, Q389R, S397T, L468I, and G512Q.

In some embodiments, the DNA polymerase mutant comprises any one of the following mutations or any combination thereof relative to the wild-type polymerase, thereby improving its thermal stability at room temperature:
Y161L, M198A, L318N, Q313C, S319K, E162L, S24R, I67A, M114T, T210D, and E233K. In some embodiments, the DNA polymerase mutant comprises any one of the following mutations or any combination thereof relative to the wild-type polymerase, thereby increasing its enzyme-template binding ability: K491R, K116R, K315R, K316R, S319C, N317K, S319R, S319F.

In some embodiments, the DNA polymerase mutant comprises any one of the following mutations or any combination thereof relative to the wild-type polymerase, thereby failing to use a Mn²⁺ ion, but being able to use a Mg²⁺ ion to catalyze a polymerase synthesis reaction: L265A and N263Q.

In some embodiments, the DNA polymerase mutant comprises any one of the following mutations or any combination thereof relative to the wild-type polymerase, thereby increasing its extension rate and extension activity under the condition of high salt: E493K, D516K, N151C, T384S, L265A, Q193C, I502L, E509V, H518L, T243V.

In some embodiments, the DNA polymerase mutant comprises any one of the following mutations or any combination thereof relative to the wild-type polymerase, thereby improving DNA polymerase extension processivity: D516K, N151C, T384S, T384N, Q193C, I502L, L265A, D516A, Q193S, N151V, D516Y, D516G, D516N, N151L, Q193L, E509V, D516T, H518L, E493K, N151Q, D516S, N151C, T243S, K491R, and T243V.

In some embodiments, the DNA polymerase mutant comprises two or more of the following mutations relative to the wild-type polymerase: K116R, K315R, K316R, N317R, S319F, L265A, L265Y, E378Q, T384K, Q389R, K491R, E493Q, E493K, L468I, G512Q, D516R, T272S, T272Y, A273K, T371D, Y372F, I379K, L398T, M377Y, L398S, Y161L, M198A, L318N, Q313C, S268A, T272S, N263Q, H394I, F81I, F81V, and E30A.

In some embodiments, the DNA polymerase mutant comprises any one of the following mutation combinations relative to the wild-type polymerase:
1) M198A+E378Q+N263Q+F460I+H518R;
2) M198A+Y372S+T384K+E493K+N263Q;
3) M198A+Y372S+T384K+K491R+G44Q;
4) L318N+M198A+T384K+E378Q+F81V;
5) E30A+L318N+E378Q+T384K+Q389R;
6) L265A+M114V+T384K+G512Q+T442K;
7) A273K+N317R+E378Q+K491R+I379K;
8) A273K+N317R+E378Q+E493K+I379K;
9) L265A+Q313C+E30A+H394I+E491M;
10) L265A+E378Q+T384K+K491R+E493K+F81I;
11) L265A+E378Q+T384K+K491R+E493K+F81V;
12) L265I+Q313C+E30A+H394I+K491M;
13) L265V+Q313C+E30A+H394I+E493V;
14) L265I+F219Y+K491R+K380R+I502L;
15) L265I+F81V+T384K+F219Y+I502L;
16) L265A+E378Q+T384K+K491R+E493K;
17) T272S+E378Q+F219Y+L318N+D516R;
18) G44Q+T272S+E378D+E493K+G512Q;
19) D34Q+T272Y+E378Q+M377Y+K491R;
20) T272Y+E378Q+M377Y+K491R+G512Q;
21) D34Q+Q313C+K491R+F81I+D516R;
22) G44Q+H394I+K491R+F81V+I502L;
23) T371D+E378Q+T384K+F81I+G512Q;
24) T371D+E378Q+T384K+F81V+D516R;
25) K116R+Y372F+L318N+H394I+Q313Y;
26) K116R+Y372F+L318N+H394I+Q313N;
27) S268N+Y161L+L398T+L265A+T384K;
28) L265A+E378Q+T384K+K491R+E493K+F81I+I502L;
29) L265A+E378Q+T384K+Q389R+K491R+E493Q+D516R;
30) T272Y+E378Q+M377Y+K491R+G512Q+F81I;
31) T272Y+E378Q+M377Y+K491R+G512Q+F81V;
32) T272Y+E378Q+M377Y+K491R+G512Q+F81I+I502L;
33) G44Q+T272S+E378Q+E493K+G512Q+D516R;
34) K116R+Y372F+L318N+H394I+Q313N+I502L; and
35) K116R+Y372F+L318N+H394I+Q313N+F81V.

In some embodiments, the DNA polymerase mutant comprises 1) an amino acid sequence shown in any of SEQ ID NOs: 2-36; or 2) an amino acid sequence having at least 70% amino acid sequence identity to an amino acid sequence shown in any of SEQ ID NOs: 2-36 and having a DNA polymerase activity.

In some embodiments, the DNA polymerase mutant is fused with a SpyCatcher protein.

In another aspect, provided herein is a method for sequencing a nucleic acid molecule, comprising using the above DNA polymerase mutant in a sequencing process.

In some embodiments, the method uses a sequencing-by-synthesis technology.

In some embodiments, the sequencing is single-molecule sequencing.

In some embodiments, the sequencing is single-molecule sequencing using a nanopore.

In some embodiments, the nanopore is fused with a SpyTag.

In some embodiments, the DNA polymerase mutant is conjugated to the nanopore.

In some embodiments, the method performs DNA synthesis using four types of nucleotide monomers carrying different modifying groups, so as to sequentially identify different nucleotide residues incorporated into a newly synthesized DNA single strand; preferably, the modifying groups are attached to phosphate groups of the nucleotide monomers.

In some embodiments, the sequencing-by-synthesis is performed under conditions containing 60-200 mM KCl.

In some embodiments, the sequencing-by-synthesis is performed under conditions containing Mg²⁺.

In another aspect, provided herein is a kit or a device for replication, amplification, or sequencing of a nucleic acid molecule, comprising the above DNA polymerase mutant.

In some embodiments, the sequencing is performed via a sequencing-by-synthesis technology.

In some embodiments, the sequencing is single-molecule sequencing.

In some embodiments, the sequencing is single-molecule sequencing using a nanopore.

In some embodiments, the kit or the device further comprises four types of nucleotide monomers with different modifying groups; preferably, the modifying groups are attached to phosphate groups of the nucleotide monomers.

In some embodiments, the DNA polymerase mutant is conjugated to the nanopore.

The polymerase mutants provided herein show excellent overall performance in terms of thermal stability, extension capability at room temperature, extension processivity, the ability to utilize modified nucleotide substrates, catalytic kinetics of strand-displacement function, and electrolyte tolerance and the like. They are suitable for the single-molecule sequencing technology based on the "sequencing-by-synthesis" scheme.

### Description of the Drawings

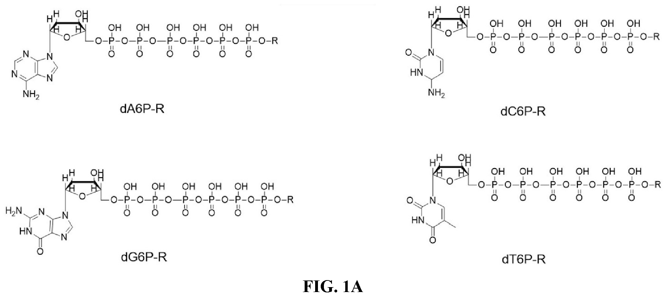

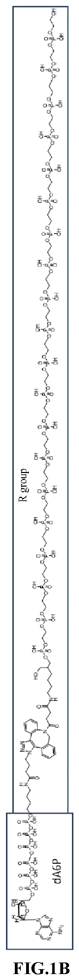

Figure 1A is a schematic diagram of the structures of dN6P-Rs, wherein the R groups are different charged modifying groups at the phosphate ends of the dN6Ps; these different modifying structures are represented by the chemically universal R. Figure 1B is a schematic diagram showing a possible R-group modification structure on dN6P. The specific modifying structure is used to interact with a nanopore to generate a different blocking current, but does not affect the catalytic kinetic properties of an enzyme.
Figure 2 shows a diagram of the structure and the sequence information of the DNA20T template.
Figures 3A and 3B show the representative results of extension rate for the polymerase wild-type (L001) and its mutants using nucleotides with modified phosphate ends.
Figures 4A, 4B, and 4C show the results of thermal stability of the DNA polymerases mutated from the polymerase wild-type (L001).
Figure 5 shows a diagram of the structure and the sequence information of the RCA88 template.
Figure 6 shows the representative results for assessments of DNA binding ability of the polymerase wild-type (L001) and its mutants.
Figure 7 shows the representative results of metal ion selectivity of the polymerase wild-type (L001) and its mutants.
Figures 8A, 8B, and 8C show the representative results of assessments of salt tolerance of the polymerase wild-type (L001) and its mutants at different concentrations of the salt KCl.
Figure 9 shows the results of extension processivity of the DNA polymerases mutated from the polymerase wild-type (L001).

### Specific Embodiments

Unless otherwise stated, all technical or scientific terms used in the present invention have the meaning as commonly understood by one of ordinary skill in the art to which the present invention pertains.

The term "or" refers to any single element of the listed alternative elements, unless the context explicitly indicates otherwise. The term "and/or" refers to any one, two, three, or more, or all of the listed alternative elements.

The terms "including", "comprising", and "having" refer to "including but not limited to", while also encompassing the case of consisting only of the listed elements.

The term "wild-type polymerase" or "polymerase w*i*ld*-*type" herein refers to the B family DNA polymerase from the bacteriophage *Actinomyces naeslundii* Phage *Av-1*, which has the amino acid sequence shown in SEQ ID NO: 1. The modifier term "wild-type" used here does not refer to that it is necessary to be naturally occurring in nature, but refers to that the wild-type polymerase herein serves as a reference for determination of the mutation(s) comprised in the polymerase mutants provided herein and the position(s) of the mutation(s), or it may be considered as the parent protein of the polymerase mutants provided herein. Additionally, in some cases, the wild-type polymerase also serves as a reference to investigate the various activities or properties of the polymerase mutants provided herein.

"Polymerase mutant" or "polymerase mutant strain" herein refers to a protein that has a difference in amino acid sequence relative to the wild-type polymerase. This difference can manifest as one or more amino acid substitution(s), deletion(s), or insertion(s) at one or more position(s) of the amino acid sequence.

"Amino acid substitution", also known as "amino acid replacement", refers to the substitution of one amino acid (referred to as original amino acid) at a particular position in an amino acid sequence by another amino acid (substituted amino acid). For example, if the amino acid at position 2 of a wild-type protein is a glutamate residue (E) and the amino acid at the corresponding position of a mutant protein is an alanine residue (A), it can be considered that there is an amino acid substitution in the mutant protein: glutamate substituted with alanine at position 2. For amino acid substitution, the following nomenclature is used herein: original amino acid, position, and substituted amino acid, with single-letter abbreviations specified by IUPAC used for amino acid names. For the example described above, the substitution can be expressed as E2A. When a mutant protein comprises two or more amino acid substitutions at the same time, it can be considered that a combination of amino acid substitutions is present therein. "Combination of amino acid substitutions" herein refers to the presence of two or more positions with amino acid substitutions in a mutant protein. For example, if the amino acid at position 2 of a wild-type protein is a glutamate residue (E) and the amino acid at the corresponding position of a mutant protein is an asparagine residue (N), and at the same time, the amino acid at position 23 of the wild-type protein is a glutamate residue (E) and the amino acid at the corresponding position of the mutant protein is an alanine residue (A), then it can be considered that there is a combination of amino acid substitutions in the mutant protein: E2N and E23A. When multiple amino acid substitutions are present in a certain mutant at the same time, the multiple mutations can be separated by "+". For example, "E2N+E23A+Y65R" represents substitutions of glutamate with asparagine, glutamate with alanine, and tyrosine with arginine at positions 2, 23, and 65, respectively.

"Amino acid sequence" herein is synonymous with the term "polypeptide", "protein", or "peptide", and they can be used interchangeably. An amino acid residue in an amino acid sequence can be expressed using a conventional single-letter or three-letter amino acid name, wherein the amino acid sequence is presented in a standard amino-to-carboxyl terminal orientation (that is, N→C).

When referring to amino acid sequences, the term "sequence identity" (also called "sequence sameness") refers to a measure of the identity between two amino acid sequences (such as a query sequence and a reference sequence) which is usually expressed in percentage. Generally, the sequences are aligned and a gap (if any) is introduced before the percent identity between the two amino acid sequences is calculated. If the amino acid residues in the two sequences are the same at a certain position after alignment, the two sequences are considered to be identical or matched at that position; if not, they are considered to be not identical or mismatched at that position. In some algorithms, the number of positions where there is a match is divided by the total number of positions in the alignment window to obtain the sequence identity. In other algorithms, the number of gaps and/or the gap length are/is also considered. Commonly used sequence alignment algorithms or software include DANMAN, ClustalW, MAFFT, BLAST, MUSCLE, and the like. For the purposes of the present invention, the publicly available alignment software BLAST (available from https://www.ncbi.nlm.nih.gov/) can be used to obtain an optimal sequence alignment and calculate the sequence identity between two amino acid sequences by using default settings.

The present inventors selected the gene fragment corresponding to the amino acid sequence of SEQ ID NO: 1 to optimize biochemical properties of the polymerase and investigate the possibility of its application in a single-molecule sequencing platform. The gene fragment is derived from the bacteriophage *Actinomyces naeslundii* Phage *Av-1*, identified in the literature as corresponding to a DNA polymerase of the bacteriophage. Based on sequence alignment with other polymerases, it is speculated that the polymerase is a B family DNA polymerase. Existing documents have disclosed its polymerase activity and application in the single-molecule sequencing technology, but there is no report on its biochemical engineering or optimization of its enzymatic kinetic characteristics.

In Example 1 below, the present invention obtained the DNA polymerase of SEQ ID NO: 1 (hereinafter also referred to as "L001") and its mutant protein products through recombinant expression in *Escherichia coli*, followed by purification using affinity chromatography and molecular sieve methods.

In Example 2, the purified protein was subjected to a DNA extension-based experiment, which demonstrated that the polymerase corresponding to the gene fragment had extension activity. With the protocol, it is also proved that, in addition to the extension activity, the polymerase further had a strand displacement function. In the process of extending the product strand at the 3' end, the polymerase could unwind the original double-stranded structure of the template-complementary strands and substituted the original complementary strand with the newly synthesized product strand, thereby forming a new double-stranded structure of template-product strands. In Example 2, the polymerase corresponding to L001 used four types of nucleotides with modified phosphate ends for effective extension. In this embodiment, the modified nucleotides could interact with the nanopores to give blocking currents specific for the base types, which was the basis for nanopore-based detection to achieve single-molecule sequencing during synthesis of a nucleic acid chain. In this embodiment, if nucleotides of any certain base type (A, T, C, or G) were omitted in a corresponding synthesis reaction for SEQ ID NO: 1, sustained extension reactions could not be completed with the polymerase. The results showed that when a particular base type was required for a synthesis reaction according to the template sequence, nucleotides of other base types could not be used for the reaction by the polymerase corresponding to L001 and its mutants. The particular nucleotide modifications could be effectively used, but did not alter the base-type specificity requirements for the polymerase. This is the biochemical basis for use of the polymerase with these particular modified nucleotides to support single-molecule sequencing. After structural engineering, the DNA polymerases comprising at least one mutation selected from the following mutations increased the extension rate with the modified nucleotides: T272S, T272Y, A273K, T371D, Y372F, L265A, I379K, L398T, M377Y, E378Q, T384K, L398S, E493K, E493Q, K491R, Q389R, S397T, L468I, G512Q, thereby increasing the throughput of single-molecule sequencing.

In Example 3, the present invention further determined that the polymerase corresponding to L001 had certain activity at room temperature. This means that the polymerase could be compatible with a nanopore-based detection platform. As a membrane protein, a nanopore needed to be embedded in a bilayer structure similar to a cell membrane to function properly. Such a membrane environment usually required a temperature near room temperature to be present stably. The polymerases identified by the present invention could exhibit certain extension and strand displacement activities at a temperature near room temperature. After structural engineering, the DNA polymerases comprising at least one mutation selected from the following mutations increased their thermal stability at room temperature: Y161L, M198A, L318N, Q313C, S319K, E162L, S24R, I67A, M114T, T210D, or E233K. The obtained mutants showed prospects of their application in the single-molecule sequencing technology combined with nanopore-based detection.

In Example 4, in the sequencing model of sequencing-by-synthesis, the formation of a polymerase-template DNA complex was a prerequisite for synthesis and sequencing. The wild-type polymerase L001 exhibited abilities of binding to the DNA circular template, capturing the correct nucleotides, and displacing the strand for extension. In a single-molecule sequencing platform, a DNA polymerase having a high ability of binding to a template usually could reduce the template concentration requirements for sequencing, which is conducive to obtaining a higher sensitivity in detection. The DNA polymerases comprising at least one mutation selected from the following mutations increased their enzyme-template binding ability: K491R, K116R, K315R, K316R, S319C, N317K, S319R, or S319F, demonstrating better prospects for their application in sequencing.

In Example 5, when modified nucleotides were used for extension, the wild-type polymerase L001 could only use Mn²⁺ as an essential catalytic ion, while the mutant with the mutation L265A, N263Q could not use a Mn²⁺ ion, but could use a Mg²⁺ ion to catalyze the corresponding synthesis reactions. Generally, when a polymerase coordinated with Mg²⁺, there would be higher selectivity for nucleotide base types, higher fidelity, and reduced interference signals during sequencing, which facilitated obtaining higher sequencing accuracy. Therefore, compared with the wild-type polymerase, the mutant with the mutation L265A, N263Q acquired the property of using Mg²⁺ for catalysis with modified nucleotides, demonstrating better prospects for its application in sequencing.

In a nanopore-based sequencing scheme, a particular variation in electrical current is the signal basis for sequencing that can be achieved. Therefore, a certain concentration of electrolyte within the entire sequencing system was required to generate sufficient current intensity to support a signal-to-noise ratio for base identification. Generally, an electrolyte concentration which supported sequencing was higher than the ideal electrolyte concentration for polymerase activity. Therefore, the present invention further investigated the extension activity and extension processivity of the polymerase corresponding to L001 at possible electrolyte concentrations for sequencing. In Example 6, different KCl concentrations were used in detection of polymerase activity, including 60 mM, 100 mM, 120 mM, 160 mM, 180 mM, 200 mM, and the like. The results showed that the extension rate of the polymerase L001 mutant was decreased by 80% under the condition of 200 mM KCl compared with 60 mM KCl, while the extension activity remained unchanged. After structural engineering, the DNA polymerases comprising at least one mutation selected from the following mutations increased their extension rate and extension activity under the condition of high salt (200 mM KCl): E493K, D516K, N151C, T384S, L265A, Q193C, I502L, E509V, H518L, or T243V, demonstrating better prospects for their application in sequencing.

For single-molecule sequencing scenarios, in addition to the focus on efficiency of a polymerase in synthesis reaction, the length of a DNA product chain that an enzyme molecule could continuously extend after binding to a template DNA at a time, that is, the processivity parameter of the polymerase, determined the sequence read length that could be obtained in sequencing, and thus was also an intense focus in the single-molecule sequencing scheme. In Example 7, in addition to the pre-formed polymerase-DNA template complex, a high concentration of interfering DNA molecules was also added to the extension system. Once the polymerase was dissociated from the DNA template, the DNA binding sites on the enzyme would be preferentially occupied by the high concentration of interfering DNA, so that the polymerase could no longer effectively bind to the DNA template molecule again to continue extension reactions. Under such conditions, the extension length achievable by the polymerase represented the length that could be obtained by continuous extension after a single event of binding to the template DNA, namely, the processivity of the polymerase under the conditions. The results showed that in an environment of 200 mM KCl and 50 µM interfering DNA, the polymerase corresponding to L001 could still produce a DNA product chain with a length of hundreds of bases. After structural engineering, the DNA polymerases comprising at least one mutation selected from the following mutations increased the extension length without affecting the extension rate: D516K, N151C, T384S, T384N, Q193C, I502L, L265A, D516A, Q193S, N151V, D516Y, D516G, D516N, N151L, Q193L, E509V, D516T, H518L, E493K, N151Q, D516S, N151C, T243S, K491R, or T243V, demonstrating the long processivity of the above mutants in high-electrolyte environment and the prospects of their application in the single-molecule sequencing technology.

The present invention discloses the various biochemical properties of the mutants of the polymerase corresponding to L001 which are closely related to single-molecule sequencing applications, and the performance of the polymerase in actual sequencing. In addition, it was found that the mutants having substitutions at multiple sites by introducing another/other amino acid substitution(s) to the polymerase could show more desirable comprehensive performance, thereby significantly increasing the read length and the accuracy of sequencing. Moreover, the present inventors also found that when a polymerase was used to achieve single-molecule sequencing during synthesis, the sequencing accuracy was often relatively low due to excessive insertion errors or deletion errors, making it difficult to turn the prospects of application in sequencing into reality. For example, the wild-type polymerase L001 identified in the present invention had a sequencing accuracy of just over 60% due to excessive Indel-type errors, which could hardly meet the requirements of sequencing applications. To find a mutant with a higher sequencing accuracy was also one of the important aspects of the present invention. For a polymerase, if the rate of the chemical reaction it catalyzed at the single-molecule level was too slow, it might result in repeated attempts of nucleotide capture and collision for a single chemical reaction step, leading to insertion errors; or if the reaction rate was too fast, the sequencing system might have difficulties in capturing a corresponding signal, leading to deletion errors. The finding of a polymerase mutant that demonstrated more favorable kinetic characteristics in single-molecule sequencing compared to the wild-type polymerase could theoretically lead to a significantly increased sequencing accuracy.

The DNA polymerases having one or more mutation(s) obtained by the present invention demonstrated superior kinetic characteristics compared to the wild-type polymerase. In terms of various sequencing-related properties, the DNA polymerase mutants comprising at least two mutations selected from the following mutations increased the accuracy and improved processivity, heat tolerance, and salt tolerance: K116R, K315R, K316R, N317R, S319F, L265A, L265Y, E378Q, T384K, Q389R, K491R, E493Q, E493K, L468I, G512Q, D516R, T272S, T272Y, A273K, T371D, Y372F, I379K, L398T, M377Y, L398S, Y161L, M198A, L318N, Q313C, S268A, T272S, N263Q, H394I, F81I, F81V, or E30A. For example, the polymerase mutant L007 (L265A+K491R+E493K+E378Q+ T384K) yielded sequencing results with continuous reads of more than 3,000 bp, and comparison with the DNA sequence to be tested showed that the sequencing accuracy could approach 85%. The polymerases reported in the present invention unequivocally showed the prospects that they could be applied to single-molecule sequencing.

In summary, a series of Examples showed that the L001 polymerase mutants identified by the present invention had excellent comprehensive performance in terms of various sequencing-related properties. The present invention provided novel polymerases that can be used for the development of sequencing technology. After subsequent mutation engineering and optimization, the present invention obtained information on mutations to improve the various biochemical properties of the polymerase. The mutants identified by the present invention showed superior performance in various aspects, having prospects of direct application in the sequencing industry.

The present invention will be described in further detail through specific Examples below. The following Examples are descriptive illustrations by way of example and are not intended to be limiting. Based on the contents and the Examples of the present invention, a person skilled in the art may obtain variations of embodiments different from the Examples mentioned in the present invention in specific implementations through reasonable natural extension, and it cannot be determined based on this that such variations exceed the scope of the present invention.

### Example 1: Cloning, expression and purification of DNA polymerase and mutants

Vector construction: The DNA sequence corresponding to SEQ ID NO: 1 (L001) was synthesized by Sangon Biotech (Shanghai) Co., Ltd., cloned and introduced to the pET26b vector (Novagen). To test the properties of the DNA polymerase on a chip, we fused a SpyCatcher protein to the DNA polymerase. In order to improve the properties of the DNA polymerase, the present invention investigated mutations at multiple amino acid sites. Mutation was constructed by designing a primer containing a mutation at a corresponding site (Sangon Biotech) and introducing the mutation at the corresponding site into the gene by PCR (phusion polymerase kit, New England Biolabs). A PCR product was purified with the Tiagene gel recovery kit (QIAquick Gel Extraction Kit) and then introduced into the vector through recombinant construction based on the sequences on the primers which were the same as those of the vector. The plasmid was transformed into a competent DH5a *E. coli* cell by chemical transformation. After a single clone was used for sequencing (Sangon Biotech) and the sequence was validated, the plasmid was extracted and stored at -20°C, and the strain was stored at -80°C.

Protein expression: The plasmid was transformed into a competent BL21 (DE3) *E. coli* cell by chemical transformation. A single colony was picked from a plate, transferred into 4 mL of LB medium containing kanamycin, and cultured at 37°C and 220 RPM overnight. A seed culture was inoculated into a new resistance medium at a ratio of 1:100, subjected to scale-up cultivation (250 mL, 1,000 mL), added with an inducer (IPTG = 250 µM) at the OD value (1.5), and cultured at 16°C and 220 RPM overnight for expression. 10-50 µL of bacterial culture was collected, centrifuged to remove the supernatant, and added with 1xbugbuster (BugBuster^{®} 10X Protein Extraction Reagent, Milipore) for cell lysis. A Sangon precast protein gel (Precast-GLgel, Hepes, SDS-PAGE, 10%, 10Well) was taken and run to detect protein expression.

Protein purification: Bacterial culture was centrifuged to collect cells. The cells were resuspended with a lysis solution (50 mM Tris-HCl (pH 8.0), 1 M NaCl, 10% glycerol, 0.1% Tween, 2 mM TCEP), and sonicated on ice for 20 min (60% power, 3 s sonication with 6 s interval). A lysate of the bacterial culture was centrifuged to collect the supernatant. The protein of interest was purified with affinity chromatography on an NI column, and further purified with molecular sieve chromatography (Superdex 200 Increase 10/300 GL). The final protein of interest was stored in the buffer (50 mM Tris7.5, 100 mM KCl, 0.1 mM EDTA, 1 mM TCEP, 0.5% Tween-20, 0.5% NP-40, 50% Glycerol) and cryopreserved at -80°C.

### Example 2: Extension rate of DNA polymerase mutants using nucleotides with modified phosphate ends

**Table 1. Reagent composition table for extension rate testing experiment**

| Name of reagent | Final concentration |
|---|---|
| DNA20T | 25 nM |
| Enzyme | 100 nM |
| Tris7.5 | 20 mM |
| KCl | 200 mM |
| dN6P-Rs (see Figures 1A and 1B for structures) | 5 uM |
| Mn²⁺ | 0.3 mM |

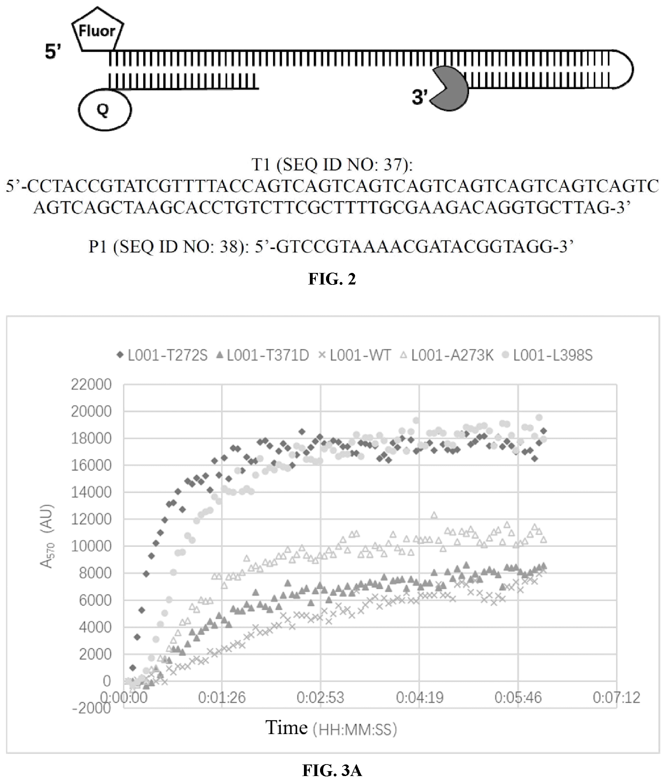

Template preparation: The schematic diagram of the DNA20T template structure was shown in Figure 2. A template chain T1 (5'-cy3) and a pointer chain P1 (3'-BHQ2) were mixed at a molar ratio of 1:1.2, heated in a metal bath at 95°C for 5 min and cooled naturally to 25°C for annealing, and the complex thus formed was named DNA20T.
Template chain T1 (SEQ ID NO: 37) 
5’-CCTACCGTATCGTTTTACCAGTCAGTCAGTCAGTCAGTCAGTCAGTCAGTC
AGTCAGCTAAGCACCTGTCTTCGCTTTTGCGAAGACAGGTGCTTAG-3’
Pointer chain P1 (SEQ ID NO: 38)
   5'-GTCCGTAAAACGATACGGTAGG-3'

Test for extension rate with modified nucleotides: Referring to the formula in Table 1, reaction was carried out in a 96-well microplate (Black 96 well cell culture plate, Flat Bottom, WHB): First, the DNA20T and the DNA polymerase or its mutants were mixed and incubated on ice for 10 min. Then, Tris, KCl, and dN6P-Rs or dNTPs were gradually added, and Mn²⁺ was injected via the sample injection pump of the microplate reader (Synergy H1, Bio-Tek). Detection was carried out under the conditions of an excitation light at 540 nm and an emission light at 570 nm. The extension rate referred to the number of nucleotides incorporated into a newly synthesized chain per unit time. In this Example, it was quantified by the increase of fluorescence value (at 570 nm) per unit time. Before extension, the fluorescent group (-Cy3) on the template and the quenching group (-BHQ2) located on the pointer chain were very close in spatial distance, thus, the fluorescent group was quenched and unable to emit fluorescence. With extension of the newly synthesized chain and the strand displacement effect of the DNA polymerase on the pointer chain, the fluorescent group was separated from the quenching group and emit fluorescence, and thus, the increase of fluorescence per unit time reflected the extension rate of the newly synthesized chain.

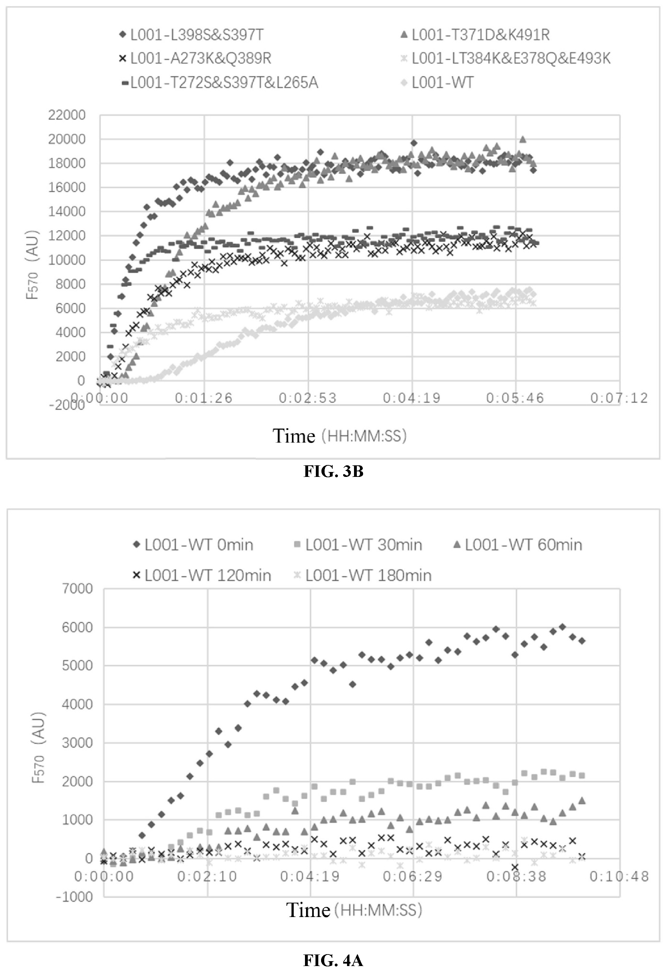

The test results showed that the DNA polymerase L001 and its mutants were able to bind to and carry out extension along the DNA template in the presence of modified nucleotides dA6P, dT6P, dC6P, and dG6P. After structural engineering, the DNA polymerase mutants comprising at least one of the following mutations increased their extension rate with the modified nucleotides: T272S, T272Y, A273K, T371D, Y372F, L265A, I379K, L398T, M377Y, E378Q, T384K, L398S, E493K, E493Q, K491R, Q389R, S397T, L468I, G512Q, or G400I (see Figures 3A and 3B, and Table 2). However, when only 3 of the 4 types of essential nucleotides were present in the reaction solution, the DNA polymerase L001 and the above mutants could not lead to extension, demonstrating that DNA polymerase L001 and the above mutants could distinguish a correct nucleotide from an incorrect nucleotide when extending the DNA chain. The template DNA20T used in the present test comprised 18 double-stranded regions and 39 single-stranded regions. The test demonstrated that the DNA polymerase L001 and its mutants had the ability of unwinding a double-stranded DNA and displacing a strand in it.

**Table 2. Mutation site and change of extension rate**

| Mutation site | Speed (S) | Speed increase fold |
|---|---|---|
| L001-WT | 108 | 1.00 |
| S397T | 32 | 3.38 |
| G512Q | 80 | 1.35 |
| K491R | 72 | 1.50 |
| Q389R | 40 | 2.70 |
| L468I | 62 | 1.74 |
| T384K | 45 | 2.40 |
| E378Q | 54 | 2.00 |
| L265A | 96 | 1.13 |
| E493Q | 36 | 3.00 |
| E493K | 48 | 2.25 |
| T272S | 20 | 5.40 |
| T272Y | 100 | 1.08 |
| A273K | 56 | 1.93 |
| T371D | 64 | 1.69 |
| Y372F | 50 | 2.16 |
| M377Y | 54 | 2.00 |
| I379K | 50 | 2.16 |
| L398S | 44 | 2.45 |
| L398T | 96 | 1.13 |
| L398S+S397T | 21 | 5.14 |
| T371D+K491R | 54 | 2.00 |
| A273K+Q389R | 30 | 3.60 |
| Q389R+L468I | 36 | 3.00 |
| M377Y+A273K | 25 | 4.32 |
| L398S+L265A | 21 | 5.14 |
| L265A+K491R | 32 | 3.38 |
| T272S+E493Q+I379K | 18 | 6.00 |
| T384K+E378Q+E493K | 30 | 3.60 |
| T272S+S397T+L265A | 12 | 9.00 |

### Example 3: Assessment of thermal stability of the polymerase and its mutants

**Table 3. Reagent composition table for thermal stability assessment experiment**

| Name of reagent | Final concentration |
|---|---|
| DNA20T | 50 nM |
| Enzyme | 50 nM |
| Tris7.5 | 20 mM |
| KCl | 200 mM |
| dN6P-Rs | 5 uM |
| Mn²⁺ | 0.3 mM |

Reference was made to the formula in Table 3. First, an enzyme was heated. In the thermal stability test, the enzyme was heated at a temperature of 30°C using a PCR instrument. After heated for 0/30/60/120/180 min, the DNA20T and the enzyme were mixed and incubated on ice for 10 min. Then, Tris, KCl and dN6P-Rs were gradually added, and Mn²⁺ was injected via the sample injection pump of the microplate reader. Detection was carried out under the conditions of an excitation light at 540 nm and an emission light at 570 nm. Stability at a corresponding time point = (F*ₜ* - F*_{BG}*)/(F*ₜ₀* - F*_{BG}*), wherein t = 30/60/120/180 min, F*ₜ* referred to the produced fluorescence value tested after the enzyme was heated for corresponding time, F*ₜ₀* referred to the resulted fluorescence value tested after the enzyme was heated for 0 min, and F*_{BG}* referred to the background fluorescence of the system, which was the value of fluorescence produced at the detection wavelength by the reaction of the enzyme with other reagents after the enzyme was inactivated at a high temperature.

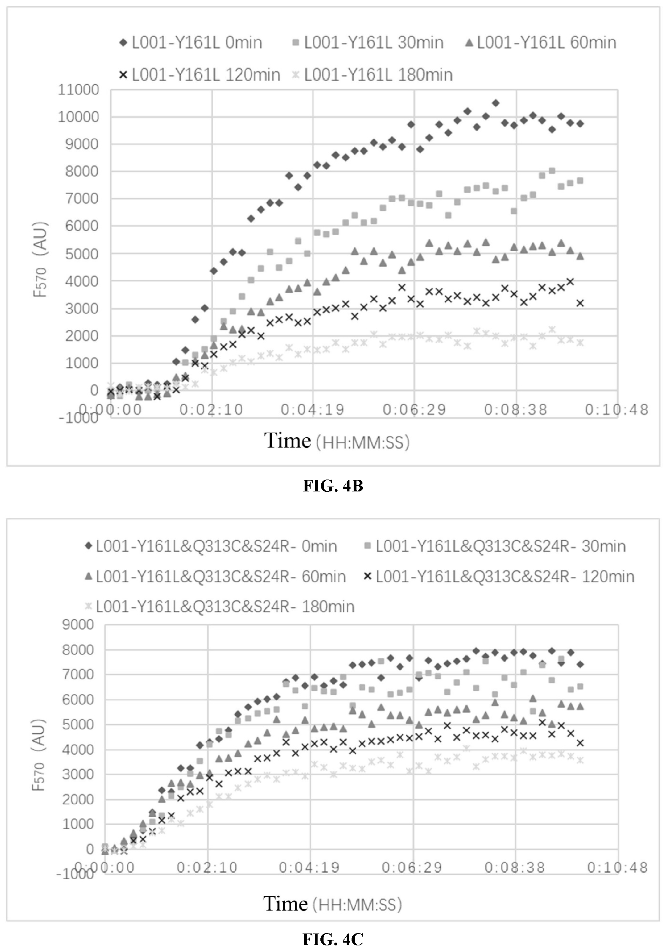

The test results showed that the DNA polymerases comprising at least one mutation selected from the following mutations increased their thermal stability at 30°C: Y161L, M198A, L318N, Q313C, S319K, E162L, S24R, I67A, M114T, T210D, or E233K (see Figures 4A-4C and Table 4).

**Table 4. Results of thermal stability of mutated DNA polymerases**

| **Percentage of residual enzyme activity** | | | | | |
|---|---|---|---|---|---|
| Mutation site | 0 min | 30 min | 60 min | 120 min | 180 min |
| L001-WT | 100% | 37% | 21% | 4% | 2% |
| S319K | 100% | 62% | 48% | 31% | 15% |
| E162L | 100% | 80% | 61% | 37% | 18% |
| L318N | 100% | 51% | 38% | 19% | 11% |
| M198A | 100% | 58% | 40% | 22% | 14% |
| Q313C | 100% | 45% | 33% | 16% | 9% |
| Y161L | 100% | 75% | 52% | 36% | 19% |
| E233K | 100% | 70% | 47% | 26% | 19% |
| I67A | 100% | 53% | 35% | 21% | 11% |
| M114T | 100% | 54% | 37% | 22% | 14% |
| S24R | 100% | 68% | 37% | 22% | 14% |
| T210D | 100% | 49% | 28% | 20% | 10% |
| E162L+S24R | 100% | 88% | 75% | 52% | 31% |
| M114T+S24R | 100% | 77% | 65% | 46% | 27% |
| Y161L+Q313C | 100% | 82% | 70% | 49% | 29% |
| L318N+M198A | 100% | 66% | 56% | 39% | 24% |
| E162L+M198A | 100% | 86% | 73% | 51% | 31% |
| E162L+S24R+E233K | 100% | 96% | 86% | 77% | 60% |
| L318N+M198A+E162L | 100% | 92% | 80% | 72% | 51% |
| Y161L+Q313C+S24R | 100% | 89% | 80% | 68% | 48% |

### Example 4: Assessment of DNA binding ability of polymerase mutants

**Table 5. Reagent composition table for DNA binding ability testing experiment**

| Name of reagent | Final concentration |
|---|---|
| RCA88 | 50 nM |
| Enzyme | 200 nM |
| Tris7.5 | 20 mM |
| KCl | 200 mM |
| dN6P-Rs | 100 uM |
| Interfering DNA | 50 uM |
| Ca²⁺ | 0.5 mM |

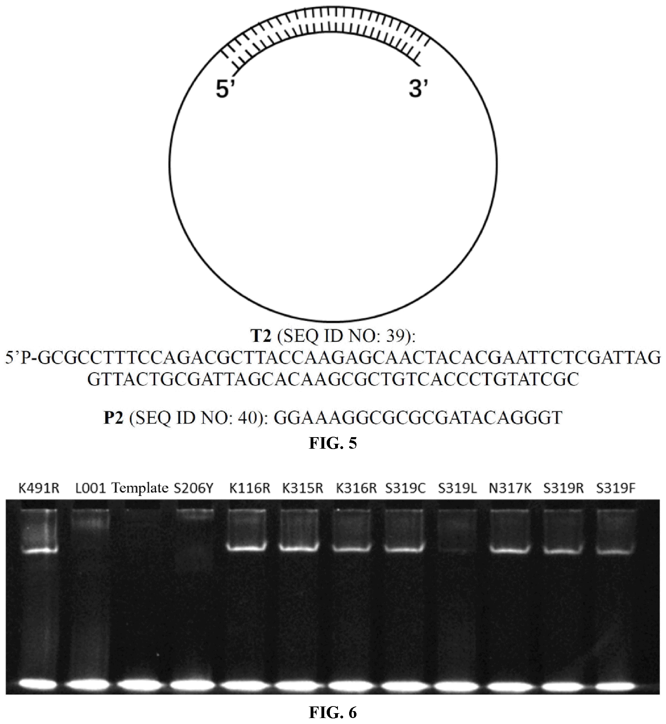

Template preparation: The structure of RCA88 was shown in Figure 5. The template chain T2 (5'-P) and the primer chain P2 were mixed at a molar ratio of 1:1.2, heated in a metal bath at 95°C for 5 min, and cooled naturally to 25°C. A T4 ligase and a buffer were added, kept at 10°C overnight for ligation, heated at 65°C for 30 min, and centrifuged at 12,000 g for 5 min. The target template, named RCA88, was collected using molecular sieve chromatography. The concentration of RCA88T was quantified based on A260 (Nanodorp).
Template chain T2 (SEQ ID NO: 39) 
5'P-GCGCCTTTCCAGACGCTTACCAAGAGCAACTACACGAATTCTCGATTAG
GTTACTGCGATTAGCACAAGCGCTGTCACCCTGTATCGC
Primer chain P2 (SEQ ID NO: 40)
   GGAAAGGCGCGCGATACAGGGT

Reference was made to the formula in Table 5. First, RCA88 and an enzyme were mixed and incubated on ice for 30 min. Then, Tris, KCl, dN6P-Rs, interfering DNA, and Ca ion were gradually added. Preparation of 1% denaturing agarose gel: 1 g of agarose was dissolved in 100 ml ddH₂O, boiled, cooled to 60°C, and added with 30 mM NaOH and 2 mM EDTA. A DNA dye (Gelred, Sangon Biotech) and a loading buffer were added to a sample, and electrophoresis (120 V, 20 min) was performed in the running buffer (50 mM Tris 8.0, 50 mM KCl, 1 mM EDTA, pH adjusted to 8.2 with KOH). The brightness of the migrated DNA bands was compared. In the simulated extension system, under the conditions of a Ca ion and DN6P-Rs, an enzyme, after binding to the template, formed a stable binary complex not available for extension. In the presence of a 1,000-fold excess of interfering DNA, an enzyme with weak DNA-binding ability would, after dissociation, rapidly bind to the interfering DNA which was in a far excessive amount, and could no longer rebind to the template. The final binary complex formed by the enzyme and the template migrated slower in the denaturing agarose gel, and the band of the complex was larger than that of the template. The brightness of the DNA band with slower migration reflected the amount of the binary complex formed by the enzyme binding to the template.

The test results showed that the DNA polymerases comprising at least one mutation selected from the following mutations increased their enzyme-template binding ability: K491R, K116R, K315R, K316R, S319C, N317K, S319R, or S319F. These polymerases could increase the amount of the enzyme-template binary complex, indicating that these mutations increased the template binding ability of the DNA polymerases (see Figure 6). Moreover, in Figure 6, no obvious band formed by the wild-type L001 binding to the template was seen, indicating that the content of the complex formed by L001 binding to the template was low, or the enzyme had a weak DNA binding ability, which led to rapid dissociation of the binary complex in the presence of interfering DNA under the electrophoresis conditions, ultimately resulting in an amount of the complex retained after electrophoresis below the detection limit under the conditions of this experiment.

### Example 5: Assessment of catalytic ion selectivity of polymerase mutants

**Table 6. Reagent composition table for metal ion selectivity assessment experiment**

| Name of reagent | Final concentration |
|---|---|
| DNA20T | 25 nM |
| Enzyme | 100 nM |
| Tris7.5 | 20 mM |
| KCl | 200 mM |
| dN6P-Rs | 5 uM |
| Mn²⁺/Mg²⁺ | 0.3 mM/10 mM |

Reference was made to the formula in Table 6. Reaction was carried out in a 96-well microplate. First, DNA20T and an enzyme were mixed and incubated on ice for 10 min. Then, Tris, KCl and dN6P-Rs were gradually added, and Mn²⁺ or Mg²⁺ was injected via the sample injection pump of the microplate reader. Detection was carried out under the conditions of an excitation light at 540 nm and an emission light at 570 nm.

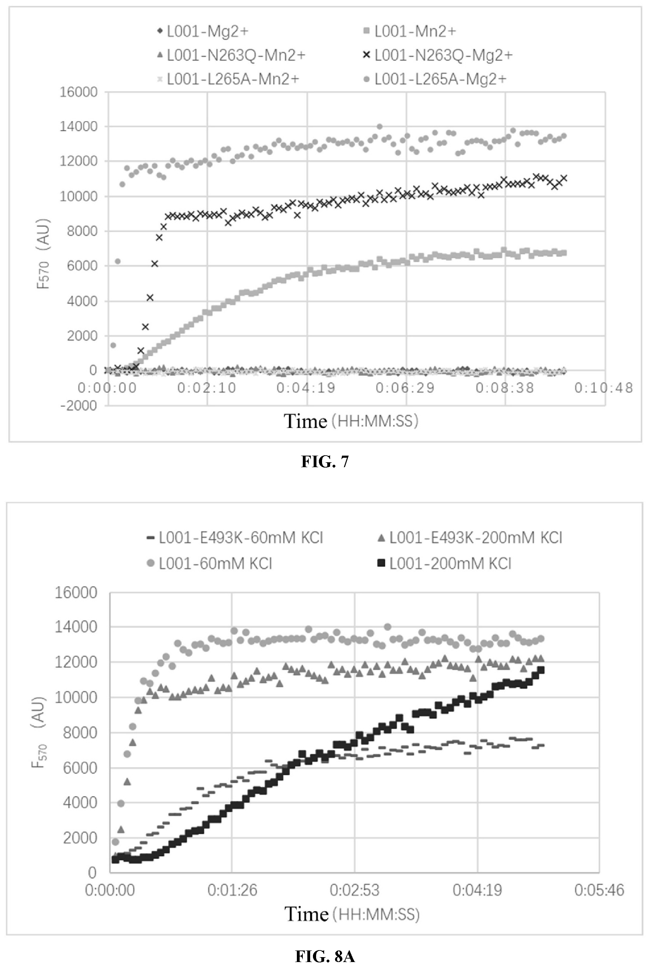

The test results showed that the formation of the quaternary complex of enzyme-template-metal ion-modified nucleotide was a necessary condition for the progression of extension. The preference of the mutants for a metal ion (Mn²⁺/Mg²⁺) was reflected in whether the extension reaction could proceed normally and the extension rate being faster or slower when Mn²⁺ or Mg²⁺ was added to the reaction solution. The DNA polymerase L001 could use modified bases for normal extension under the condition of Mn²⁺, but not under the condition of Mg²⁺. The metal ion preference of the mutant with the mutation site L265A, N263Q was opposite to that of the wild type, wherein extension was not available under the condition of Mn²⁺, but the mutant could use modified bases for normal extension under the condition of Mg²⁺ (see Figure 7).

### Example 6: Assessment of salt tolerance of polymerase mutants

**Table 7. Reagent composition table for salt tolerance assessment experiment**

| Name of reagent | Final concentration |
|---|---|
| DNA20T | 50 nM |
| Enzyme | 200 nM |
| Tris7.5 | 20 mM |
| KCl | 60/100/120/160/180/200 mM |
| dN6P-Rs | 100 uM |
| Interfering DNA | 50 uM |
| Mn²⁺ | 0.3 mM |

Reference was made to the formula in Table 7. First, DNA20T and an enzyme were mixed and incubated on ice for 30 min. The salt concentration for the salt tolerance test was 60/100/120/160/180/200 mM of KCl. Then, Tris, KCl, dN6P-Rs and interfering DNA were gradually added, and Mn²⁺ was injected via the sample injection pump of the microplate reader. Detection was carried out under the conditions of an excitation light at 540 nm and an emission light at 570 nm. The time required for fluorescence to reach equilibrium upon reaction completion reflected the extension rate of the enzyme. An enzyme formed a binary complex with the template. At varying salt concentrations, an enzyme with weak DNA-binding affinity would, after dissociation, rapidly bind to the interfering DNA in a far excessive amount, and could no longer rebind to the template. The fluorescence signal generated upon reaction completion directly reflected the extension activity of an enzyme.

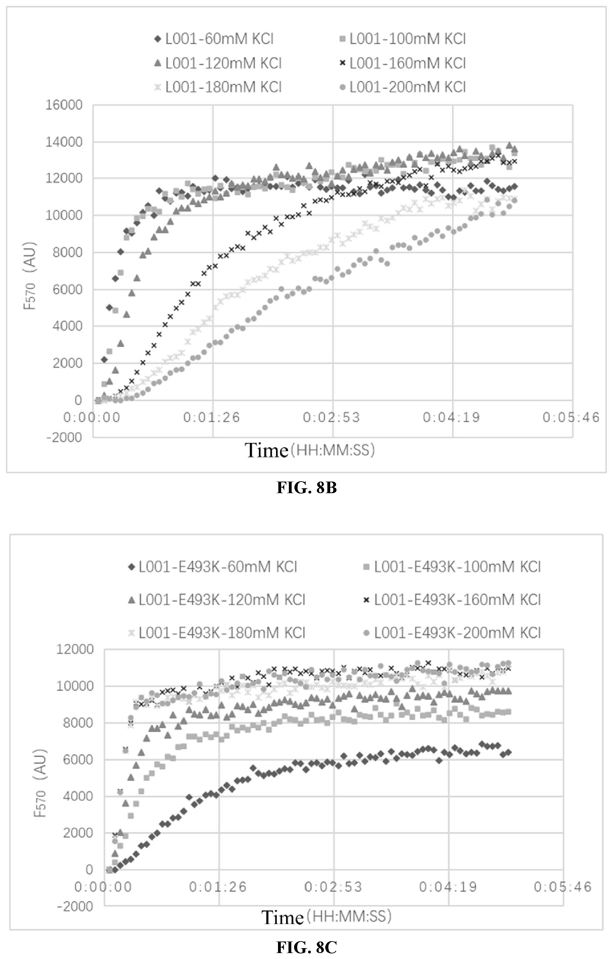

The test results showed that in the presence of the interfering DNA, the extension rate of the polymerase L001 mutant was decreased by 80% under the condition of 200 mM KCl compared with 60 mM KCl, while the extension activity remained unchanged. After structural engineering, the DNA polymerases comprising at least one mutation selected from the following mutations increased their extension rate and extension activity under the condition of high salt (200 mM KCl): E493K, D516K, N151C, T384S, L265A, Q193C, I502L, E509V, H518L, or T243V (see Tables 8 and 9). Among these polymerases, the mutant with E493K, under the condition of 200 mM KCl, could still effectively catalyze a synthesis reaction, with the reaction completed in about 32 s and a fluorescence signal generated (about 10,064). The extension rate was increased by 200% and the extension activity was increased by 60% compared to those under the condition of 60 mM KCl, with the reaction completed in about 100 s and a fluorescence signal generated (about 6,000) (see Figures 8A-8C).

**Table 8. Results of extension rate of polymerases with different mutation sites at different KCl concentrations**

| **Speed (S)** | | | | | | |
|---|---|---|---|---|---|---|
| Mutation site | 60 mM KCl | 100 mM KCl | 120 mM KCl | 160 mM KCl | 180 mM KCl | 200 mM KCl |
| L001-WT | 18 | 18 | 28 | 64 | 88 | 112 |
| D516K | 80 | 48 | 28 | 16 | 13 | 13 |
| E493K | 52 | 26 | 18 | 12 | 12 | 12 |
| E509V | 119 | 60 | 36 | 24 | 16 | 12 |
| H518L | 78 | 40 | 25 | 15 | 12 | 12 |
| I502L | 116 | 62 | 36 | 20 | 10 | 10 |
| L265A | 114 | 58 | 30 | 15 | 12 | 12 |
| N151C | 65 | 24 | 10 | 5 | 5 | 5 |
| Q193C | 111 | 70 | 52 | 33 | 18 | 15 |
| T243V | 52 | 20 | 6 | 4 | 4 | 4 |
| T384S | 88 | 45 | 30 | 16 | 12 | 12 |
| E493K+H518L | 68 | 36 | 20 | 10 | 5 | 5 |
| H518L+L265A | 68 | 40 | 22 | 12 | 6 | 6 |
| N151C+T384S | 60 | 34 | 18 | 10 | 8 | 8 |
| D516K+I502L | 75 | 40 | 22 | 12 | 12 | 12 |
| E493K+Q193C | 68 | 36 | 18 | 10 | 8 | 8 |
| T243V+E509V | 48 | 28 | 15 | 6 | 6 | 6 |
| E493K+D516K+T243V | 72 | 38 | 20 | 12 | 10 | 10 |
| E493K+D516K+L265A | 59 | 32 | 15 | 10 | 8 | 8 |
| E493K+D516K+Q193C | 40 | 20 | 12 | 6 | 6 | 6 |
| D516K+I502L+T243V | 50 | 24 | 12 | 6 | 6 | 6 |

**Table 9. Results of extension activity of polymerases with different mutation sites at different KCl concentrations**

| **Fluorescence signal (RFU)** | | | | | | |
|---|---|---|---|---|---|---|
| Mutation site | 60 mM KCl | 100 mM KCl | 120 mM KCl | 160 mM KCl | 180 mM KCl | 200 mM KCl |
| L001-WT | 11478 | 13167 | 13331 | 12867 | 10796 | 10219 |
| D516K | 7268 | 8201 | 9005 | 9623 | 9520 | 9681 |
| E493K | 6525 | 8530 | 9593 | 10772 | 10677 | 11001 |
| E509V | 9234 | 9857 | 10710 | 10909 | 10907 | 10843 |
| H518L | 9734 | 9972 | 10893 | 10976 | 10971 | 10865 |
| I502L | 8965 | 9415 | 10514 | 10542 | 10869 | 10634 |
| L265A | 8935 | 9382 | 10300 | 10374 | 10794 | 10601 |
| N151C | 8785 | 9285 | 10293 | 10317 | 10781 | 10584 |
| Q193C | 8718 | 9269 | 9787 | 10278 | 10711 | 10514 |
| T243V | 7119 | 7287 | 9040 | 9786 | 10241 | 10248 |
| T384S | 7435 | 7619 | 9117 | 9957 | 10256 | 10276 |
| E493K+H518L | 7940 | 7881 | 9194 | 9974 | 10297 | 10312 |
| H518L+L265A | 8051 | 8087 | 9286 | 9980 | 10332 | 10343 |
| N151C+T384S | 8258 | 8199 | 9458 | 10152 | 10434 | 10416 |
| D516K+I502L | 8432 | 8,353 | 9552 | 10229 | 10502 | 10417 |
| E493K+Q193C | 8775 | 9271 | 10114 | 10317 | 10765 | 10517 |
| T243V+E509V | 6165 | 7210 | 9012 | 9371 | 9990 | 10205 |
| E493K+D516K+T243V | 6488 | 7255 | 9038 | 9471 | 10071 | 10247 |
| E493K+D516K+L265A | 5144 | 6082 | 8353 | 9066 | 9676 | 10032 |
| E493K+D516K+Q193C | 5302 | 6104 | 8718 | 9215 | 9803 | 10108 |
| D516K+I502L+T243V | 5378 | 6196 | 8947 | 9230 | 9938 | 10195 |

### Example 7: Assessment of processivity of polymerase mutants

**Table 10. Reagent composition table for processivity assessment experiment**

| Name of reagent | Final concentration |
|---|---|
| RCA88 | 50 nM |
| Enzyme | 200 nM |
| Tris7.5 | 20 mM |
| KCl | 200 mM |
| dN6P-Rs | 100 uM |
| Interfering DNA | 50 uM |
| Mn²⁺ | 0.3 mM |

Reference was made to the formula in Table 10. First, RCA88 and an enzyme were mixed and incubated on ice for 30 min. Then, Tris, KCl, dN6P-Rs and interfering DNA were gradually added, kept at 28°C for extension for 1 h, and heated at 95°C for 10 min to stop reactions. Preparation of 1% denaturing agarose gel: 1 g of agarose was dissolved in 100 ml ddH₂O, boiled, cooled to 60°C, and added with 30 mM NaOH and 2 mM EDTA. A DNA dye (Gelred, Sangon Biotech) and a loading buffer were added to a sample, and electrophoresis (120 V, 20 min) was performed in the running buffer (50 mM Tris 8.0, 50 mM KCl, 1 mM EDTA, pH adjusted to 8.2 with KOH). The brightness and length of the DNA bands were compared. An enzyme formed a binary complex with the template for extension under the actions of a Mn ion and DN6P-Rs. In the process of extension, an enzyme with poor processivity would, after dissociation from the template, rapidly bind to the interfering DNA in a far excessive amount, and was no longer available for rebinding or participating in extension. The final extension products on electrophoresis bands showed varying degrees of brightness and lengths, reflecting the extension processivity of the enzymes under the experimental conditions.

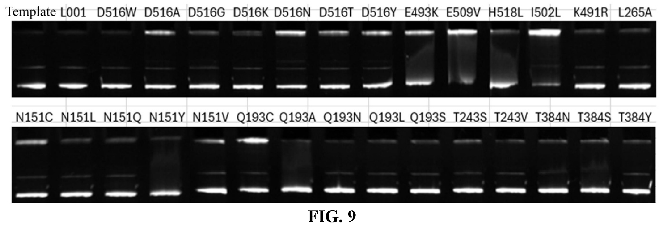

The test results showed that in the presence of the interfering DNA, the DNA polymerases comprising at least one mutation selected from the following mutations increased the extension length without affecting the extension rate under the experimental condition of 200 mM KCl: D516K, N151C, T384S, T384N, Q193C, I502L, L265A, D516A, Q193S, N151V, D516Y, D516G, D516N, N151L, Q193L, E509V, D516T, H518L, E493K, N151Q, D516S, N151C, T243S, K491R, or T243V (see Figure 9), thereby improving the enzyme processivity.

### Example 8: Assessment of performance of polymerase mutants in sequencing applications

The DNA polymerase and its mutants were conjugated to the protein nanopore by means of protein fusion. The α-hemolysin pore heptamer fused with a SpyTag and the DNA polymerase or its mutants fused with a SpyCatcher were mixed in a molar ratio of 1:2, and left at room temperature for 30 min. The conjugation efficiency was determined via electrophoresis.

After conjugation, the complex (template-polymerase-α-hemolysin) was embedded into a lipid bilayer above an etched sequencing unit pore on a semiconductor sensor chip. Dynamic capture of modified nucleotides by the enzyme-template-nanopore complex was recorded at 120 mV/-180 mV. Based on the current blockades induced by the modified nucleotides upon capture by the DNA polymerases, the types of the modified nucleotides, blocking frequency, dwell time and other information were determined. Comparison was made with the template sequence to assess the kinetic characteristics of the DNA polymerases.

The test results showed that the DNA polymerases comprising at least two or more mutations selected from the following mutations increased the accuracy and improved processivity, heat tolerance, and salt tolerance: D516R, E30A, E378D, E378Q, E491M, E493K, E493Q, E493V, F219Y, F460I, F81I, F81V, G44Q, G512Q, H394I, H518R, I379K, I502L, K380R, K491M, K491R, L265A, L318N, L398T, M114V, M198A, M377Y, N263Q, N317R, Q313C, Q313N, Q313Y, Q389R, T272S, T272Y, T384K, T442K, Y161L, Y372F, or Y372S. Specifically, the polymerase L027 (K116R+Y372F+L318N+H394I+Q313N) yielded sequencing results with continuous reads of more than 3,000 bp, and comparison with the DNA sequence to be tested showed that the sequencing accuracy could approach 85%. Table 11 showed the characteristic data of some representative mutants in sequencing applications.

**Table 11. Characteristic data of representative mutants in sequencing applications**

| Sequence ID | Mutant | Median read length (bps) | Median accuracy (%) |
|---|---|---|---|
| NO: 1 | L001 | 580 | 67 |
| NO: 2 | M198A+E378Q+N263Q+F460I+H518R | 2453 | 80 |
| NO: 3 | M198A+Y372S+T384K+E493K+N263Q | 1980 | 88 |
| NO: 4 | M198A+Y372S+T384K+K491R+G44Q | 3576 | 82 |
| NO: 5 | L318N+M198A+T384K+E378Q+F81V | 1817 | 86 |
| NO: 6 | E30A+L318N+E378Q+T384K+Q389R | 3812 | 83 |
| NO: 7 | L265A+M114V+T384K+G512Q+T442K | 2624 | 83 |
| NO: 8 | A273K+N317R+E378Q+K491R+I379K | 4700 | 81 |
| NO: 9 | A273K+N317R+E378Q+E493K+I379K | 3751 | 80 |
| NO: 10 | L265A+Q313C+E30A+H394I+E491M | 2825 | 83 |
| NO: 11 | L265A+E378Q+T384K+K491R+E493K+F81I | 1237 | 83 |
| NO: 12 | L265A+E378Q+T384K+K491R+E493K+F81V | 1415 | 83 |
| NO: 13 | L265I+Q313C+E30A+H394I+K491M | 3124 | 83 |
| NO: 14 | L265V+Q313C+E30A+H394I+E493V | 1675 | 84 |
| NO: 15 | L265I+F219Y+K491R+K380R+I502L | 2125 | 83 |
| NO: 16 | L265I+F81V+T384K+F219Y+I502L | 2536 | 84 |
| NO: 17 | L265A+E378Q+T384K+K491R+E493K | 2885 | 83 |
| NO: 18 | T272S+E378Q+F219Y+L318N+D516R | 2436 | 84 |
| NO: 19 | G44Q+T272S+E378D+E493K+G512Q | 2735 | 84 |
| NO: 20 | D34Q+T272Y+E378Q+M377Y+K491R | 1988 | 83 |
| NO: 21 | T272Y+E378Q+M377Y+K491R+G512Q | 2113 | 88 |
| NO: 22 | D34Q+Q313C+K491R+F81I+D516R | 3553 | 82 |
| NO: 23 | G44Q+H394I+K491R+F81V+I502L | 3246 | 82 |
| NO: 24 | T371D+E378Q+T384K+F81I+G512Q | 1688 | 83 |
| NO: 25 | T371D+E378Q+T384K+F81V+D516R | 2412 | 83 |
| NO: 26 | K116R+Y372F+L318N+H394I+Q313Y | 2845 | 84 |
| NO: 27 | K116R+Y372F+L318N+H394I+Q313N | 3252 | 85 |
| NO: 28 | S268N+Y161L+L398T+L265A+T384K | 2250 | 82 |
| NO: 29 | L265A+E378Q+T384K+K491R+E493K+F81I+I502L | 2823 | 80 |
| NO: 30 | L265A+E378Q+T384K+Q389R+K491R+E493Q+D516R | 2325 | 81 |
| NO: 31 | T272Y+E378Q+M377Y+K491R+G512Q+F81I | 1879 | 84 |
| NO: 32 | T272Y+E378Q+M377Y+K491R+G512Q+F81V | 2620 | 80 |
| NO: 33 | T272Y+E378Q+M377Y+K491R+G512Q+F81I+I502L | 2220 | 83 |
| NO: 34 | G44Q+T272S+E378Q+E493K+G512Q+D516R | 2652 | 83 |
| NO: 35 | K116R+Y372F+L318N+H394I+Q313N+I502L | 2725 | 85 |
| NO: 36 | K116R+Y372F+L318N+H394I+Q313N+F81V | 2925 | 83 |

The present invention is directed to a single-molecule sequencing scheme based on the combination of a nanopore and sequencing-by-synthesis, identifies a novel nucleic acid polymerase, and, through subsequent in-depth optimization and engineering, obtains polymerases with improved comprehensive performance, which support the use with a nanopore-based detection platform to obtain high-quality sequencing data, and thus have industrialization prospects.

The sequences of the polymerase L001 and some of the polymerase mutants mentioned herein are as follows.

| Sequen ce ID | Amino acid sequence |
|---|---|
| NO: 1 | MVRQSTIASPARGGVRRSHKKVPSFCADFETTTDEDDCRVWSWGIIQVGKLQNYV DGISLDGFMSHISERASHIYFHNLAFDGTFILDWLLKHGYRWTKENPGVKEFTSL ISRMGKYYSITVVFETGFRVEFRDSFKKLPMSVSAIAKAFNLHDQKLEIDYEKPR PIGYIPTEQEKRYQRNDVAIVAQALEVQFAEKMTKLTAGSDSLATYKKMTGKLFI RRFPILSPEIDTEIRKAYRGGFTYADPRYAKKLNGKGSVYDVNSLYPSVMRTALL PYGEPIYSEGAPRTNRPLYIASITFTAKLKPNHIPCIQIKKNLSFNPTQYLEEVK EPTTVVATNIDIELWKKHYDFKIYSWNGTFEFRGSHGFFDTYVDHFMEIKKNSTG GLRQIAKLHLNSLYGKFATNPDITGKHPTLKDNRVSLVMNEPETRDPVYTPMGVF ITAYARKKTISAAQDNYETFAYADTDSLHLIGPTTPPDSLWVDPVELGAWKHESS FTKSVYIRAKQYAEEIGGKLDVHIAGMPRNVAATLTLEDMLHGGTWNGKLIPVRV PGGTVLKDTTFTLKID |
| NO: 2 | MVRQSTIASPARGGVRRSHKKVPSFCADFETTTDEDDCRVWSWGIIQVGKLQNYV DGISLDGFMSHISERASHIYFHNLAFDGTFILDWLLKHGYRWTKENPGVKEFTSL ISRMGKYYSITVVFETGFRVEFRDSFKKLPMSVSAIAKAFNLHDQKLEIDYEKPR PIGYIPTEQEKRYQRNDVAIVAQALEVQFAEKATKLTAGSDSLATYKKMTGKLFI RRFPILSPEIDTEIRKAYRGGFTYADPRYAKKLNGKGSVYDVQSLYPSVMRTALL PYGEPIYSEGAPRTNRPLYIASITFTAKLKPNHIPCIQIKKNLSFNPTQYLEEVK EPTTVVATNIDIELWKKHYDFKIYSWNGTFEFRGSHGFFDTYVDHFMQIKKNSTG GLRQIAKLHLNSLYGKFATNPDITGKHPTLKDNRVSLVMNEPETRDPVYTPMGVF ITAYARKKTISAAQDNYETIAYADTDSLHLIGPTTPPDSLWVDPVELGAWKHESS FTKSVYIRAKQYAEEIGGKLDVRIAGMPRNVAATLTLEDMLHGGTWNGKLIPVRV PGGTVLKDTTFTLKID |
| NO: 3 | MVRQSTIASPARGGVRRSHKKVPSFCADFETTTDEDDCRVWSWGIIQVGKLQNYV DGISLDGFMSHISERASHIYFHNLAFDGTFILDWLLKHGYRWTKENPGVKEFTSL ISRMGKYYSITVVFETGFRVEFRDSFKKLPMSVSAIAKAFNLHDQKLEIDYEKPR PIGYIPTEQEKRYQRNDVAIVAQALEVQFAEKATKLTAGSDSLATYKKMTGKLFI RRFPILSPEIDTEIRKAYRGGFTYADPRYAKKLNGKGSVYDVQSLYPSVMRTALL PYGEPIYSEGAPRTNRPLYIASITFTAKLKPNHIPCIQIKKNLSFNPTQYLEEVK EPTTVVATNIDIELWKKHYDFKIYSWNGTFEFRGSHGFFDTSVDHFMEIKKNSKG GLRQIAKLHLNSLYGKFATNPDITGKHPTLKDNRVSLVMNEPETRDPVYTPMGVF ITAYARKKTISAAQDNYETFAYADTDSLHLIGPTTPPDSLWVDPVELGAWKHESS FTKSVYIRAKQYAEEIGGKLDVHIAGMPRNVAATLTLEDMLHGGTWNGKLIPVRV PGGTVLKDTTFTLKID |
| NO: 4 | MVRQSTIASPARGGVRRSHKKVPSFCADFETTTDEDDCRVWSWQIIQVGKLQNYV DGISLDGFMSHISERASHIYFHNLAFDGTFILDWLLKHGYRWTKENPGVKEFTSL ISRMGKYYSITVVFETGFRVEFRDSFKKLPMSVSAIAKAFNLHDQKLEIDYEKPR PIGYIPTEQEKRYQRNDVAIVAQALEVQFAEKATKLTAGSDSLATYKKMTGKLFI RRFPILSPEIDTEIRKAYRGGFTYADPRYAKKLNGKGSVYDVNSLYPSVMRTALL PYGEPIYSEGAPRTNRPLYIASITFTAKLKPNHIPCIQIKKNLSFNPTQYLEEVK EPTTVVATNIDIELWKKHYDFKIYSWNGTFEFRGSHGFFDTSVDHFMEIKKNSKG GLRQIAKLHLNSLYGKFATNPDITGKHPTLKDNRVSLVMNEPETRDPVYTPMGVF |
| | ITAYARKKTISAAQDNYETFAYADTDSLHLIGPTTPPDSLWVDPVELGAWRHESS FTKSVYIRAKQYAEEIGGKLDVHIAGMPRNVAATLTLEDMLHGGTWNGKLIPVRV PGGTVLKDTTFTLKID |
| NO: 5 | MVRQSTIASPARGGVRRSHKKVPSFCADFETTTDEDDCRVWSWGIIQVGKLQNYV DGISLDGFMSHISERASHIYFHNLAVDGTFILDWLLKHGYRWTKENPGVKEFTSL ISRMGKYYSITVVFETGFRVEFRDSFKKLPMSVSAIAKAFNLHDQKLEIDYEKPR PIGYIPTEQEKRYQRNDVAIVAQALEVQFAEKATKLTAGSDSLATYKKMTGKLFI RRFPILSPEIDTEIRKAYRGGFTYADPRYAKKLNGKGSVYDVNSLYPSVMRTALL PYGEPIYSEGAPRTNRPLYIASITFTAKLKPNHIPCIQIKKNNSFNPTQYLEEVK EPTTVVATNIDIELWKKHYDFKIYSWNGTFEFRGSHGFFDTYVDHFMQIKKNSKG GLRQIAKLHLNSLYGKFATNPDITGKHPTLKDNRVSLVMNEPETRDPVYTPMGVF ITAYARKKTISAAQDNYETFAYADTDSLHLIGPTTPPDSLWVDPVELGAWKHESS FTKSVYIRAKQYAEEIGGKLDVHIAGMPRNVAATLTLEDMLHGGTWNGKLIPVRV PGGTVLKDTTFTLKID |
| NO: 6 | MVRQSTIASPARGGVRRSHKKVPSFCADFATTTDEDDCRVWSWGIIQVGKLQNYV DGISLDGFMSHISERASHIYFHNLAFDGTFILDWLLKHGYRWTKENPGVKEFTSL ISRMGKYYSITVVFETGFRVEFRDSFKKLPMSVSAIAKAFNLHDQKLEIDYEKPR PIGYIPTEQEKRYQRNDVAIVAQALEVQFAEKMTKLTAGSDSLATYKKMTGKLFI RRFPILSPEIDTEIRKAYRGGFTYADPRYAKKLNGKGSVYDVNSLYPSVMRTALL PYGEPIYSEGAPRTNRPLYIASITFTAKLKPNHIPCIQIKKNNSFNPTQYLEEVK EPTTVVATNIDIELWKKHYDFKIYSWNGTFEFRGSHGFFDTYVDHFMQIKKNSKG GLRRIAKLHLNSLYGKFATNPDITGKHPTLKDNRVSLVMNEPETRDPVYTPMGVF ITAYARKKTISAAQDNYETFAYADTDSLHLIGPTTPPDSLWVDPVELGAWKHESS FTKSVYIRAKQYAEEIGGKLDVHIAGMPRNVAATLTLEDMLHGGTWNGKLIPVRV PGGTVLKDTTFTLKID |
| NO: 7 | MVRQSTIASPARGGVRRSHKKVPSFCADFETTTDEDDCRVWSWGIIQVGKLQNYV DGISLDGFMSHISERASHIYFHNLAFDGTFILDWLLKHGYRWTKENPGVKEFTSL ISRVGKYYSITVVFETGFRVEFRDSFKKLPMSVSAIAKAFNLHDQKLEIDYEKPR PIGYIPTEQEKRYQRNDVAIVAQALEVQFAEKMTKLTAGSDSLATYKKMTGKLFI RRFPILSPEIDTEIRKAYRGGFTYADPRYAKKLNGKGSVYDVNSAYPSVMRTALL PYGEPIYSEGAPRTNRPLYIASITFTAKLKPNHIPCIQIKKNLSFNPTQYLEEVK EPTTVVATNIDIELWKKHYDFKIYSWNGTFEFRGSHGFFDTYVDHFMEIKKNSKG GLRQIAKLHLNSLYGKFATNPDITGKHPTLKDNRVSLVMNEPETRDPVYTPMGVF IKAYARKKTISAAQDNYETFAYADTDSLHLIGPTTPPDSLWVDPVELGAWKHESS FTKSVYIRAKQYAEEIQGKLDVHIAGMPRNVAATLTLEDMLHGGTWNGKLIPVRV PGGTVLKDTTFTLKID |
| NO: 8 | MVRQSTIASPARGGVRRSHKKVPSFCADFETTTDEDDCRVWSWGIIQVGKLQNYV DGISLDGFMSHISERASHIYFHNLAFDGTFILDWLLKHGYRWTKENPGVKEFTSL ISRMGKYYSITVVFETGFRVEFRDSFKKLPMSVSAIAKAFNLHDQKLEIDYEKPR PIGYIPTEQEKRYQRNDVAIVAQALEVQFAEKMTKLTAGSDSLATYKKMTGKLFI RRFPILSPEIDTEIRKAYRGGFTYADPRYAKKLNGKGSVYDVNSLYPSVMRTKLL PYGEPIYSEGAPRTNRPLYIASITFTAKLKPNHIPCIQIKKRLSFNPTQYLEEVK EPTTVVATNIDIELWKKHYDFKIYSWNGTFEFRGSHGFFDTYVDHFMQKKKNSTG GLRQIAKLHLNSLYGKFATNPDITGKHPTLKDNRVSLVMNEPETRDPVYTPMGVF ITAYARKKTISAAQDNYETFAYADTDSLHLIGPTTPPDSLWVDPVELGAWRHESS FTKSVYIRAKQYAEEIGGKLDVHIAGMPRNVAATLTLEDMLHGGTWNGKLIPVRV PGGTVLKDTTFTLKID |
| NO: 9 | MVRQSTIASPARGGVRRSHKKVPSFCADFETTTDEDDCRVWSWGIIQVGKLQNYV DGISLDGFMSHISERASHIYFHNLAFDGTFILDWLLKHGYRWTKENPGVKEFTSL ISRMGKYYSITVVFETGFRVEFRDSFKKLPMSVSAIAKAFNLHDQKLEIDYEKPR PIGYIPTEQEKRYQRNDVAIVAQALEVQFAEKMTKLTAGSDSLATYKKMTGKLFI RRFPILSPEIDTEIRKAYRGGFTYADPRYAKKLNGKGSVYDVNSLYPSVMRTKLL |
| | PYGEPIYSEGAPRTNRPLYIASITFTAKLKPNHIPCIQIKKRLSFNPTQYLEEVK EPTTVVATNIDIELWKKHYDFKIYSWNGTFEFRGSHGFFDTYVDHFMQKKKNSTG GLRQIAKLHLNSLYGKFATNPDITGKHPTLKDNRVSLVMNEPETRDPVYTPMGVF ITAYARKKTISAAQDNYETFAYADTDSLHLIGPTTPPDSLWVDPVELGAWKHKSS FTKSVYIRAKQYAEEIGGKLDVHIAGMPRNVAATLTLEDMLHGGTWNGKLIPVRV PGGTVLKDTTFTLKID |
| NO: 10 | MVRQSTIASPARGGVRRSHKKVPSFCADFATTTDEDDCRVWSWGIIQVGKLQNYV DGISLDGFMSHISERASHIYFHNLAFDGTFILDWLLKHGYRWTKENPGVKEFTSL ISRMGKYYSITVVFETGFRVEFRDSFKKLPMSVSAIAKAFNLHDQKLEIDYEKPR PIGYIPTEQEKRYQRNDVAIVAQALEVQFAEKMTKLTAGSDSLATYKKMTGKLFI RRFPILSPEIDTEIRKAYRGGFTYADPRYAKKLNGKGSVYDVNSAYPSVMRTALL PYGEPIYSEGAPRTNRPLYIASITFTAKLKPNHIPCICIKKNLSFNPTQYLEEVK EPTTVVATNIDIELWKKHYDFKIYSWNGTFEFRGSHGFFDTYVDHFMEIKKNSTG GLRQIAKLILNSLYGKFATNPDITGKHPTLKDNRVSLVMNEPETRDPVYTPMGVF ITAYARKKTISAAQDNYETFAYADTDSLHLIGPTTPPDSLWVDPVELGAWMHESS FTKSVYIRAKQYAEEIGGKLDVHIAGMPRNVAATLTLEDMLHGGTWNGKLIPVRV PGGTVLKDTTFTLKID |
| NO: 11 | MVRQSTIASPARGGVRRSHKKVPSFCADFETTTDEDDCRVWSWGIIQVGKLQNYV DGISLDGFMSHISERASHIYFHNLAIDGTFILDWLLKHGYRWTKENPGVKEFTSL ISRMGKYYSITVVFETGFRVEFRDSFKKLPMSVSAIAKAFNLHDQKLEIDYEKPR PIGYIPTEQEKRYQRNDVAIVAQALEVQFAEKMTKLTAGSDSLATYKKMTGKLFI RRFPILSPEIDTEIRKAYRGGFTYADPRYAKKLNGKGSVYDVNSAYPSVMRTALL PYGEPIYSEGAPRTNRPLYIASITFTAKLKPNHIPCIQIKKNLSFNPTQYLEEVK EPTTVVATNIDIELWKKHYDFKIYSWNGTFEFRGSHGFFDTYVDHFMQIKKNSKG GLRQIAKLHLNSLYGKFATNPDITGKHPTLKDNRVSLVMNEPETRDPVYTPMGVF ITAYARKKTISAAQDNYETFAYADTDSLHLIGPTTPPDSLWVDPVELGAWRHKSS FTKSVYIRAKQYAEEIGGKLDVHIAGMPRNVAATLTLEDMLHGGTWNGKLIPVRV PGGTVLKDTTFTLKID |
| NO: 12 | MVRQSTIASPARGGVRRSHKKVPSFCADFETTTDEDDCRVWSWGIIQVGKLQNYV DGISLDGFMSHISERASHIYFHNLAVDGTFILDWLLKHGYRWTKENPGVKEFTSL ISRMGKYYSITVVFETGFRVEFRDSFKKLPMSVSAIAKAFNLHDQKLEIDYEKPR PIGYIPTEQEKRYQRNDVAIVAQALEVQFAEKMTKLTAGSDSLATYKKMTGKLFI RRFPILSPEIDTEIRKAYRGGFTYADPRYAKKLNGKGSVYDVNSAYPSVMRTALL PYGEPIYSEGAPRTNRPLYIASITFTAKLKPNHIPCIQIKKNLSFNPTQYLEEVK EPTTVVATNIDIELWKKHYDFKIYSWNGTFEFRGSHGFFDTYVDHFMQIKKNSKG GLRQIAKLHLNSLYGKFATNPDITGKHPTLKDNRVSLVMNEPETRDPVYTPMGVF ITAYARKKTISAAQDNYETFAYADTDSLHLIGPTTPPDSLWVDPVELGAWRHKSS FTKSVYIRAKQYAEEIGGKLDVHIAGMPRNVAATLTLEDMLHGGTWNGKLIPVRV PGGTVLKDTTFTLKID |
| NO: 13 | MVRQSTIASPARGGVRRSHKKVPSFCADFATTTDEDDCRVWSWGIIQVGKLQNYV DGISLDGFMSHISERASHIYFHNLAFDGTFILDWLLKHGYRWTKENPGVKEFTSL ISRMGKYYSITVVFETGFRVEFRDSFKKLPMSVSAIAKAFNLHDQKLEIDYEKPR PIGYIPTEQEKRYQRNDVAIVAQALEVQFAEKMTKLTAGSDSLATYKKMTGKLFI RRFPILSPEIDTEIRKAYRGGFTYADPRYAKKLNGKGSVYDVNSIYPSVMRTALL PYGEPIYSEGAPRTNRPLYIASITFTAKLKPNHIPCICIKKNLSFNPTQYLEEVK EPTTVVATNIDIELWKKHYDFKIYSWNGTFEFRGSHGFFDTYVDHFMEIKKNSTG GLRQIAKLILNSLYGKFATNPDITGKHPTLKDNRVSLVMNEPETRDPVYTPMGVF ITAYARKKTISAAQDNYETFAYADTDSLHLIGPTTPPDSLWVDPVELGAWMHESS FTKSVYIRAKQYAEEIGGKLDVHIAGMPRNVAATLTLEDMLHGGTWNGKLIPVRV PGGTVLKDTTFTLKID |
| NO: 14 | MVRQSTIASPARGGVRRSHKKVPSFCADFATTTDEDDCRVWSWGIIQVGKLQNYV DGISLDGFMSHISERASHIYFHNLAFDGTFILDWLLKHGYRWTKENPGVKEFTSL |
| | ISRMGKYYSITVVFETGFRVEFRDSFKKLPMSVSAIAKAFNLHDQKLEIDYEKPR PIGYIPTEQEKRYQRNDVAIVAQALEVQFAEKMTKLTAGSDSLATYKKMTGKLFI RRFPILSPEIDTEIRKAYRGGFTYADPRYAKKLNGKGSVYDVNSVYPSVMRTALL PYGEPIYSEGAPRTNRPLYIASITFTAKLKPNHIPCICIKKNLSFNPTQYLEEVK EPTTVVATNIDIELWKKHYDFKIYSWNGTFEFRGSHGFFDTYVDHFMEIKKNSTG GLRQIAKLILNSLYGKFATNPDITGKHPTLKDNRVSLVMNEPETRDPVYTPMGVF ITAYARKKTISAAQDNYETFAYADTDSLHLIGPTTPPDSLWVDPVELGAWKHVSS FTKSVYIRAKQYAEEIGGKLDVHIAGMPRNVAATLTLEDMLHGGTWNGKLIPVRV PGGTVLKDTTFTLKID |
| NO: 15 | MVRQSTIASPARGGVRRSHKKVPSFCADFETTTDEDDCRVWSWGIIQVGKLQNYV DGISLDGFMSHISERASHIYFHNLAFDGTFILDWLLKHGYRWTKENPGVKEFTSL ISRMGKYYSITVVFETGFRVEFRDSFKKLPMSVSAIAKAFNLHDQKLEIDYEKPR PIGYIPTEQEKRYQRNDVAIVAQALEVQFAEKMTKLTAGSDSLATYKKMTGKLYI RRFPILSPEIDTEIRKAYRGGFTYADPRYAKKLNGKGSVYDVNSIYPSVMRTALL PYGEPIYSEGAPRTNRPLYIASITFTAKLKPNHIPCIQIKKNLSFNPTQYLEEVR EPTTVVATNIDIELWKKHYDFKIYSWNGTFEFRGSHGFFDTYVDHFMEIKKNSTG GLRQIAKLHLNSLYGKFATNPDITGKHPTLKDNRVSLVMNEPETRDPVYTPMGVF ITAYARKKTISAAQDNYETFAYADTDSLHLIGPTTPPDSLWVDPVELGAWRHESS FTKSVYLRAKQYAEEIGGKLDVHIAGMPRNVAATLTLEDMLHGGTWNGKLIPVRV PGGTVLKDTTFTLKID |
| NO: 16 | MVRQSTIASPARGGVRRSHKKVPSFCADFETTTDEDDCRVWSWGIIQVGKLQNYV DGISLDGFMSHISERASHIYFHNLAVDGTFILDWLLKHGYRWTKENPGVKEFTSL ISRMGKYYSITVVFETGFRVEFRDSFKKLPMSVSAIAKAFNLHDQKLEIDYEKPR PIGYIPTEQEKRYQRNDVAIVAQALEVQFAEKMTKLTAGSDSLATYKKMTGKLYI RRFPILSPEIDTEIRKAYRGGFTYADPRYAKKLNGKGSVYDVNSIYPSVMRTALL PYGEPIYSEGAPRTNRPLYIASITFTAKLKPNHIPCIQIKKNLSFNPTQYLEEVK EPTTVVATNIDIELWKKHYDFKIYSWNGTFEFRGSHGFFDTYVDHFMEIKKNSKG GLRQIAKLHLNSLYGKFATNPDITGKHPTLKDNRVSLVMNEPETRDPVYTPMGVF ITAYARKKTISAAQDNYETFAYADTDSLHLIGPTTPPDSLWVDPVELGAWKHESS FTKSVYLRAKQYAEEIGGKLDVHIAGMPRNVAATLTLEDMLHGGTWNGKLIPVRV PGGTVLKDTTFTLKID |
| NO: 17 | MVRQSTIASPARGGVRRSHKKVPSFCADFETTTDEDDCRVWSWGIIQVGKLQNYV DGISLDGFMSHISERASHIYFHNLAFDGTFILDWLLKHGYRWTKENPGVKEFTSL ISRMGKYYSITVVFETGFRVEFRDSFKKLPMSVSAIAKAFNLHDQKLEIDYEKPR PIGYIPTEQEKRYQRNDVAIVAQALEVQFAEKMTKLTAGSDSLATYKKMTGKLFI RRFPILSPEIDTEIRKAYRGGFTYADPRYAKKLNGKGSVYDVNSAYPSVMRTALL PYGEPIYSEGAPRTNRPLYIASITFTAKLKPNHIPCIQIKKNLSFNPTQYLEEVK EPTTVVATNIDIELWKKHYDFKIYSWNGTFEFRGSHGFFDTYVDHFMQIKKNSKG GLRQIAKLHLNSLYGKFATNPDITGKHPTLKDNRVSLVMNEPETRDPVYTPMGVF ITAYARKKTISAAQDNYETFAYADTDSLHLIGPTTPPDSLWVDPVELGAWRHKSS FTKSVYIRAKQYAEEIGGKLDVHIAGMPRNVAATLTLEDMLHGGTWNGKLIPVRV PGGTVLKDTTFTLKID |
| NO: 18 | MVRQSTIASPARGGVRRSHKKVPSFCADFETTTDEDDCRVWSWGIIQVGKLQNYV DGISLDGFMSHISERASHIYFHNLAFDGTFILDWLLKHGYRWTKENPGVKEFTSL ISRMGKYYSITVVFETGFRVEFRDSFKKLPMSVSAIAKAFNLHDQKLEIDYEKPR PIGYIPTEQEKRYQRNDVAIVAQALEVQFAEKMTKLTAGSDSLATYKKMTGKLYI RRFPILSPEIDTEIRKAYRGGFTYADPRYAKKLNGKGSVYDVNSLYPSVMRSALL PYGEPIYSEGAPRTNRPLYIASITFTAKLKPNHIPCIQIKKNNSFNPTQYLEEVK EPTTVVATNIDIELWKKHYDFKIYSWNGTFEFRGSHGFFDTYVDHFMQIKKNSTG GLRQIAKLHLNSLYGKFATNPDITGKHPTLKDNRVSLVMNEPETRDPVYTPMGVF ITAYARKKTISAAQDNYETFAYADTDSLHLIGPTTPPDSLWVDPVELGAWKHESS FTKSVYIRAKQYAEEIGGKLRVHIAGMPRNVAATLTLEDMLHGGTWNGKLIPVRV |
| | PGGTVLKDTTFTLKID |
| NO: 19 | MVRQSTIASPARGGVRRSHKKVPSFCADFETTTDEDDCRVWSWQIIQVGKLQNYV DGISLDGFMSHISERASHIYFHNLAFDGTFILDWLLKHGYRWTKENPGVKEFTSL ISRMGKYYSITVVFETGFRVEFRDSFKKLPMSVSAIAKAFNLHDQKLEIDYEKPR PIGYIPTEQEKRYQRNDVAIVAQALEVQFAEKMTKLTAGSDSLATYKKMTGKLFI RRFPILSPEIDTEIRKAYRGGFTYADPRYAKKLNGKGSVYDVNSLYPSVMRSALL PYGEPIYSEGAPRTNRPLYIASITFTAKLKPNHIPCIQIKKNLSFNPTQYLEEVK EPTTVVATNIDIELWKKHYDFKIYSWNGTFEFRGSHGFFDTYVDHFMDIKKNSTG GLRQIAKLHLNSLYGKFATNPDITGKHPTLKDNRVSLVMNEPETRDPVYTPMGVF ITAYARKKTISAAQDNYETFAYADTDSLHLIGPTTPPDSLWVDPVELGAWKHKSS FTKSVYIRAKQYAEEIQGKLDVHIAGMPRNVAATLTLEDMLHGGTWNGKLIPVRV PGGTVLKDTTFTLKID |
| NO: 20 | MVRQSTIASPARGGVRRSHKKVPSFCAQFETTTDEDDCRVWSWGIIQVGKLQNYV DGISLDGFMSHISERASHIYFHNLAFDGTFILDWLLKHGYRWTKENPGVKEFTSL ISRMGKYYSITVVFETGFRVEFRDSFKKLPMSVSAIAKAFNLHDQKLEIDYEKPR PIGYIPTEQEKRYQRNDVAIVAQALEVQFAEKMTKLTAGSDSLATYKKMTGKLFI RRFPILSPEIDTEIRKAYRGGFTYADPRYAKKLNGKGSVYDVNSLYPSVMRYALL PYGEPIYSEGAPRTNRPLYIASITFTAKLKPNHIPCIQIKKNLSFNPTQYLEEVK EPTTVVATNIDIELWKKHYDFKIYSWNGTFEFRGSHGFFDTYVDHFYQIKKNSTG GLRQIAKLHLNSLYGKFATNPDITGKHPTLKDNRVSLVMNEPETRDPVYTPMGVF ITAYARKKTISAAQDNYETFAYADTDSLHLIGPTTPPDSLWVDPVELGAWRHESS FTKSVYIRAKQYAEEIGGKLDVHIAGMPRNVAATLTLEDMLHGGTWNGKLIPVRV PGGTVLKDTTFTLKID |
| NO: 21 | MVRQSTIASPARGGVRRSHKKVPSFCADFETTTDEDDCRVWSWGIIQVGKLQNYV DGISLDGFMSHISERASHIYFHNLAFDGTFILDWLLKHGYRWTKENPGVKEFTSL ISRMGKYYSITVVFETGFRVEFRDSFKKLPMSVSAIAKAFNLHDQKLEIDYEKPR PIGYIPTEQEKRYQRNDVAIVAQALEVQFAEKMTKLTAGSDSLATYKKMTGKLFI RRFPILSPEIDTEIRKAYRGGFTYADPRYAKKLNGKGSVYDVNSLYPSVMRYALL PYGEPIYSEGAPRTNRPLYIASITFTAKLKPNHIPCIQIKKNLSFNPTQYLEEVK EPTTVVATNIDIELWKKHYDFKIYSWNGTFEFRGSHGFFDTYVDHFYQIKKNSTG GLRQIAKLHLNSLYGKFATNPDITGKHPTLKDNRVSLVMNEPETRDPVYTPMGVF ITAYARKKTISAAQDNYETFAYADTDSLHLIGPTTPPDSLWVDPVELGAWRHESS FTKSVYIRAKQYAEEIQGKLDVHIAGMPRNVAATLTLEDMLHGGTWNGKLIPVRV PGGTVLKDTTFTLKID |
| NO: 22 | MVRQSTIASPARGGVRRSHKKVPSFCAQFETTTDEDDCRVWSWGIIQVGKLQNYV DGISLDGFMSHISERASHIYFHNLAIDGTFILDWLLKHGYRWTKENPGVKEFTSL ISRMGKYYSITVVFETGFRVEFRDSFKKLPMSVSAIAKAFNLHDQKLEIDYEKPR PIGYIPTEQEKRYQRNDVAIVAQALEVQFAEKMTKLTAGSDSLATYKKMTGKLFI RRFPILSPEIDTEIRKAYRGGFTYADPRYAKKLNGKGSVYDVNSLYPSVMRTALL PYGEPIYSEGAPRTNRPLYIASITFTAKLKPNHIPCICIKKNLSFNPTQYLEEVK EPTTVVATNIDIELWKKHYDFKIYSWNGTFEFRGSHGFFDTYVDHFMEIKKNSTG GLRQIAKLHLNSLYGKFATNPDITGKHPTLKDNRVSLVMNEPETRDPVYTPMGVF ITAYARKKTISAAQDNYETFAYADTDSLHLIGPTTPPDSLWVDPVELGAWRHESS FTKSVYIRAKQYAEEIGGKLRVHIAGMPRNVAATLTLEDMLHGGTWNGKLIPVRV PGGTVLKDTTFTLKID |
| NO: 23 | MVRQSTIASPARGGVRRSHKKVPSFCADFETTTDEDDCRVWSWQIIQVGKLQNYV DGISLDGFMSHISERASHIYFHNLAVDGTFILDWLLKHGYRWTKENPGVKEFTSL ISRMGKYYSITVVFETGFRVEFRDSFKKLPMSVSAIAKAFNLHDQKLEIDYEKPR PIGYIPTEQEKRYQRNDVAIVAQALEVQFAEKMTKLTAGSDSLATYKKMTGKLFI RRFPILSPEIDTEIRKAYRGGFTYADPRYAKKLNGKGSVYDVNSLYPSVMRTALL PYGEPIYSEGAPRTNRPLYIASITFTAKLKPNHIPCIQIKKNLSFNPTQYLEEVK EPTTVVATNIDIELWKKHYDFKIYSWNGTFEFRGSHGFFDTYVDHFMEIKKNSTG |
| | GLRQIAKLILNSLYGKFATNPDITGKHPTLKDNRVSLVMNEPETRDPVYTPMGVF ITAYARKKTISAAQDNYETFAYADTDSLHLIGPTTPPDSLWVDPVELGAWRHESS FTKSVYLRAKQYAEEIGGKLDVHIAGMPRNVAATLTLEDMLHGGTWNGKLIPVRV PGGTVLKDTTFTLKID |
| NO: 24 | MVRQSTIASPARGGVRRSHKKVPSFCADFETTTDEDDCRVWSWGIIQVGKLQNYV DGISLDGFMSHISERASHIYFHNLAIDGTFILDWLLKHGYRWTKENPGVKEFTSL ISRMGKYYSITVVFETGFRVEFRDSFKKLPMSVSAIAKAFNLHDQKLEIDYEKPR PIGYIPTEQEKRYQRNDVAIVAQALEVQFAEKMTKLTAGSDSLATYKKMTGKLFI RRFPILSPEIDTEIRKAYRGGFTYADPRYAKKLNGKGSVYDVNSLYPSVMRTALL PYGEPIYSEGAPRTNRPLYIASITFTAKLKPNHIPCIQIKKNLSFNPTQYLEEVK EPTTVVATNIDIELWKKHYDFKIYSWNGTFEFRGSHGFFDDYVDHFMQIKKNSKG GLRQIAKLHLNSLYGKFATNPDITGKHPTLKDNRVSLVMNEPETRDPVYTPMGVF ITAYARKKTISAAQDNYETFAYADTDSLHLIGPTTPPDSLWVDPVELGAWKHESS FTKSVYIRAKQYAEEIQGKLDVHIAGMPRNVAATLTLEDMLHGGTWNGKLIPVRV PGGTVLKDTTFTLKID |
| NO: 25 | MVRQSTIASPARGGVRRSHKKVPSFCADFETTTDEDDCRVWSWGIIQVGKLQNYV DGISLDGFMSHISERASHIYFHNLAIDGTFILDWLLKHGYRWTKENPGVKEFTSL ISRMGKYYSITVVFETGFRVEFRDSFKKLPMSVSAIAKAFNLHDQKLEIDYEKPR PIGYIPTEQEKRYQRNDVAIVAQALEVQFAEKMTKLTAGSDSLATYKKMTGKLFI RRFPILSPEIDTEIRKAYRGGFTYADPRYAKKLNGKGSVYDVNSLYPSVMRTALL PYGEPIYSEGAPRTNRPLYIASITFTAKLKPNHIPCIQIKKNLSFNPTQYLEEVK EPTTVVATNIDIELWKKHYDFKIYSWNGTFEFRGSHGFFDDYVDHFMQIKKNSKG GLRQIAKLHLNSLYGKFATNPDITGKHPTLKDNRVSLVMNEPETRDPVYTPMGVF ITAYARKKTISAAQDNYETFAYADTDSLHLIGPTTPPDSLWVDPVELGAWKHESS FTKSVYIRAKQYAEEIDGKLRVHIAGMPRNVAATLTLEDMLHGGTWNGKLIPVRV PGGTVLKDTTFTLKID |
| NO: 26 | MVRQSTIASPARGGVRRSHKKVPSFCADFETTTDEDDCRVWSWGIIQVGKLQNYV DGISLDGFMSHISERASHIYFHNLAFDGTFILDWLLKHGYRWTKENPGVKEFTSL ISRMGRYYSITVVFETGFRVEFRDSFKKLPMSVSAIAKAFNLHDQKLEIDYEKPR PIGYIPTEQEKRYQRNDVAIVAQALEVQFAEKMTKLTAGSDSLATYKKMTGKLFI RRFPINSPEIDTEIRKAYRGGFTYADPRYAKKLNGKGSVYDVNSLYPSVMRTALL PYGEPIYSEGAPRTNRPLYIASITFTAKLKPNHIPCIYIKKNLSFNPTQYLEEVK EPTTVVATNIDIELWKKHYDFKIYSWNGTFEFRGSHGFFDTFVDHFMEIKKNSTG GLRQIAKLILNSLYGKFATNPDITGKHPTLKDNRVSLVMNEPETRDPVYTPMGVF ITAYARKKTISAAQDNYETFAYADTDSLHLIGPTTPPDSLWVDPVELGAWKHESS FTKSVYIRAKQYAEEIGGKLDVHIAGMPRNVAATLTLEDMLHGGTWNGKLIPVRV PGGTVLKDTTFTLKID |
| NO: 27 | MVRQSTIASPARGGVRRSHKKVPSFCADFETTTDEDDCRVWSWGIIQVGKLQNYV DGISLDGFMSHISERASHIYFHNLAFDGTFILDWLLKHGYRWTKENPGVKEFTSL ISRMGRYYSITVVFETGFRVEFRDSFKKLPMSVSAIAKAFNLHDQKLEIDYEKPR PIGYIPTEQEKRYQRNDVAIVAQALEVQFAEKMTKLTAGSDSLATYKKMTGKLFI RRFPILSPEIDTEIRKAYRGGFTYADPRYAKKLNGKGSVYDVNSLYPSVMRTALL PYGEPIYSEGAPRTNRPLYIASITFTAKLKPNHIPCINIKKNNSFNPTQYLEEVK EPTTVVATNIDIELWKKHYDFKIYSWNGTFEFRGSHGFFDTFVDHFMEIKKNSTG GLRQIAKLILNSLYGKFATNPDITGKHPTLKDNRVSLVMNEPETRDPVYTPMGVF ITAYARKKTISAAQDNYETFAYADTDSLHLIGPTTPPDSLWVDPVELGAWKHESS FTKSVYIRAKQYAEEIGGKLDVHIAGMPRNVAATLTLEDMLHGGTWNGKLIPVRV PGGTVLKDTTFTLKID |
| NO: 28 | MVRQSTIASPARGGVRRSHKKVPSFCADFETTTDEDDCRVWSWGIIQVGKLQNYV DGISLDGFMSHISERASHIYFHNLAFDGTFILDWLLKHGYRWTKENPGVKEFTSL ISRMGKYYSITVVFETGFRVEFRDSFKKLPMSVSAIAKAFNLHDQKLEIDLEKPR PIGYIPTEQEKRYQRNDVAIVAQALEVQFAEKMTKLTAGSDSLATYKKMTGKLFI |
| | RRFPILSPEIDTEIRKAYRGGFTYADPRYAKKLNGKGSVYDVNSAYPNVMRTALL PYGEPIYSEGAPRTNRPLYIASITFTAKLKPNHIPCIQIKKNLSFNPTQYLEEVK EPTTVVATNIDIELWKKHYDFKIYSWNGTFEFRGSHGFFDTYVDHFMEIKKNSKG GLRQIAKLHLNSTYGKFATNPDITGKHPTLKDNRVSLVMNEPETRDPVYTPMGVF ITAYARKKTISAAQDNYETFAYADTDSLHLIGPTTPPDSLWVDPVELGAWKHESS FTKSVYIRAKQYAEEIGGKLDVHIAGMPRNVAATLTLEDMLHGGTWNGKLIPVRV PGGTVLKDTTFTLKID |
| NO: 29 | MVRQSTIASPARGGVRRSHKKVPSFCADFETTTDEDDCRVWSWGIIQVGKLQNYV DGISLDGFMSHISERASHIYFHNLAIDGTFILDWLLKHGYRWTKENPGVKEFTSL ISRMGKYYSITVVFETGFRVEFRDSFKKLPMSVSAIAKAFNLHDQKLEIDYEKPR PIGYIPTEQEKRYQRNDVAIVAQALEVQFAEKMTKLTAGSDSLATYKKMTGKLFI RRFPILSPEIDTEIRKAYRGGFTYADPRYAKKLNGKGSVYDVNSAYPSVMRTALL PYGEPIYSEGAPRTNRPLYIASITFTAKLKPNHIPCIQIKKNLSFNPTQYLEEVK EPTTVVATNIDIELWKKHYDFKIYSWNGTFEFRGSHGFFDTYVDHFMQIKKNSKG GLRQIAKLHLNSLYGKFATNPDITGKHPTLKDNRVSLVMNEPETRDPVYTPMGVF ITAYARKKTISAAQDNYETFAYADTDSLHLIGPTTPPDSLWVDPVELGAWRHKSS FTKSVYLRAKQYAEEIGGKLDVHIAGMPRNVAATLTLEDMLHGGTWNGKLIPVRV PGGTVLKDTTFTLKID |
| NO: 30 | MVRQSTIASPARGGVRRSHKKVPSFCADFETTTDEDDCRVWSWGIIQVGKLQNYV DGISLDGFMSHISERASHIYFHNLAIDGTFILDWLLKHGYRWTKENPGVKEFTSL ISRMGKYYSITVVFETGFRVEFRDSFKKLPMSVSAIAKAFNLHDQKLEIDYEKPR PIGYIPTEQEKRYQRNDVAIVAQALEVQFAEKMTKLTAGSDSLATYKKMTGKLFI RRFPILSPEIDTEIRKAYRGGFTYADPRYAKKLNGKGSVYDVNSAYPSVMRTALL PYGEPIYSEGAPRTNRPLYIASITFTAKLKPNHIPCIQIKKNLSFNPTQYLEEVK EPTTVVATNIDIELWKKHYDFKIYSWNGTFEFRGSHGFFDTYVDHFMQIKKNSKG GLRRIAKLHLNSLYGKFATNPDITGKHPTLKDNRVSLVMNEPETRDPVYTPMGVF ITAYARKKTISAAQDNYETFAYADTDSLHLIGPTTPPDSLWVDPVELGAWRHQSS FTKSVYIRAKQYAEEIGGKLRVHIAGMPRNVAATLTLEDMLHGGTWNGKLIPVRV PGGTVLKDTTFTLKID |
| NO: 31 | MVRQSTIASPARGGVRRSHKKVPSFCADFETTTDEDDCRVWSWGIIQVGKLQNYV DGISLDGFMSHISERASHIYFHNLAIDGTFILDWLLKHGYRWTKENPGVKEFTSL ISRMGKYYSITVVFETGFRVEFRDSFKKLPMSVSAIAKAFNLHDQKLEIDYEKPR PIGYIPTEQEKRYQRNDVAIVAQALEVQFAEKMTKLTAGSDSLATYKKMTGKLFI RRFPILSPEIDTEIRKAYRGGFTYADPRYAKKLNGKGSVYDVNSLYPSVMRYALL PYGEPIYSEGAPRTNRPLYIASITFTAKLKPNHIPCIQIKKNLSFNPTQYLEEVK EPTTVVATNIDIELWKKHYDFKIYSWNGTFEFRGSHGFFDTYVDHFYQIKKNSTG GLRQIAKLHLNSLYGKFATNPDITGKHPTLKDNRVSLVMNEPETRDPVYTPMGVF ITAYARKKTISAAQDNYETFAYADTDSLHLIGPTTPPDSLWVDPVELGAWRHESS FTKSVYIRAKQYAEEIQGKLDVHIAGMPRNVAATLTLEDMLHGGTWNGKLIPVRV PGGTVLKDTTFTLKID |
| NO: 32 | MVRQSTIASPARGGVRRSHKKVPSFCADFETTTDEDDCRVWSWGIIQVGKLQNYV DGISLDGFMSHISERASHIYFHNLAVDGTFILDWLLKHGYRWTKENPGVKEFTSL ISRMGKYYSITVVFETGFRVEFRDSFKKLPMSVSAIAKAFNLHDQKLEIDYEKPR PIGYIPTEQEKRYQRNDVAIVAQALEVQFAEKMTKLTAGSDSLATYKKMTGKLFI RRFPILSPEIDTEIRKAYRGGFTYADPRYAKKLNGKGSVYDVNSLYPSVMRYALL PYGEPIYSEGAPRTNRPLYIASITFTAKLKPNHIPCIQIKKNLSFNPTQYLEEVK EPTTVVATNIDIELWKKHYDFKIYSWNGTFEFRGSHGFFDTYVDHFYQIKKNSTG GLRQIAKLHLNSLYGKFATNPDITGKHPTLKDNRVSLVMNEPETRDPVYTPMGVF ITAYARKKTISAAQDNYETFAYADTDSLHLIGPTTPPDSLWVDPVELGAWRHESS FTKSVYIRAKQYAEEIQGKLDVHIAGMPRNVAATLTLEDMLHGGTWNGKLIPVRV PGGTVLKDTTFTLKID |
| NO: 33 | MVRQSTIASPARGGVRRSHKKVPSFCADFETTTDEDDCRVWSWGIIQVGKLQNYV |
| | DGISLDGFMSHISERASHIYFHNLAIDGTFILDWLLKHGYRWTKENPGVKEFTSL ISRMGKYYSITVVFETGFRVEFRDSFKKLPMSVSAIAKAFNLHDQKLEIDYEKPR PIGYIPTEQEKRYQRNDVAIVAQALEVQFAEKMTKLTAGSDSLATYKKMTGKLFI RRFPILSPEIDTEIRKAYRGGFTYADPRYAKKLNGKGSVYDVNSLYPSVMRYALL PYGEPIYSEGAPRTNRPLYIASITFTAKLKPNHIPCIQIKKNLSFNPTQYLEEVK EPTTVVATNIDIELWKKHYDFKIYSWNGTFEFRGSHGFFDTYVDHFYQIKKNSTG GLRQIAKLHLNSLYGKFATNPDITGKHPTLKDNRVSLVMNEPETRDPVYTPMGVF ITAYARKKTISAAQDNYETFAYADTDSLHLIGPTTPPDSLWVDPVELGAWRHESS FTKSVYLRAKQYAEEIQGKLDVHIAGMPRNVAATLTLEDMLHGGTWNGKLIPVRV PGGTVLKDTTFTLKID |
| NO: 34 | MVRQSTIASPARGGVRRSHKKVPSFCADFETTTDEDDCRVWSWQIIQVGKLQNYV DGISLDGFMSHISERASHIYFHNLAFDGTFILDWLLKHGYRWTKENPGVKEFTSL ISRMGKYYSITVVFETGFRVEFRDSFKKLPMSVSAIAKAFNLHDQKLEIDYEKPR PIGYIPTEQEKRYQRNDVAIVAQALEVQFAEKMTKLTAGSDSLATYKKMTGKLFI RRFPILSPEIDTEIRKAYRGGFTYADPRYAKKLNGKGSVYDVNSLYPSVMRSALL PYGEPIYSEGAPRTNRPLYIASITFTAKLKPNHIPCIQIKKNLSFNPTQYLEEVK EPTTVVATNIDIELWKKHYDFKIYSWNGTFEFRGSHGFFDTYVDHFMDIKKNSTG GLRQIAKLHLNSLYGKFATNPDITGKHPTLKDNRVSLVMNEPETRDPVYTPMGVF ITAYARKKTISAAQDNYETFAYADTDSLHLIGPTTPPDSLWVDPVELGAWKHKSS FTKSVYIRAKQYAEEIQGKLRVHIAGMPRNVAATLTLEDMLHGGTWNGKLIPVRV PGGTVLKDTTFTLKID |
| NO: 35 | MVRQSTIASPARGGVRRSHKKVPSFCADFETTTDEDDCRVWSWGIIQVGKLQNYV DGISLDGFMSHISERASHIYFHNLAFDGTFILDWLLKHGYRWTKENPGVKEFTSL ISRMGRYYSITVVFETGFRVEFRDSFKKLPMSVSAIAKAFNLHDQKLEIDYEKPR PIGYIPTEQEKRYQRNDVAIVAQALEVQFAEKMTKLTAGSDSLATYKKMTGKLFI RRFPILSPEIDTEIRKAYRGGFTYADPRYAKKLNGKGSVYDVNSLYPSVMRTALL PYGEPIYSEGAPRTNRPLYIASITFTAKLKPNHIPCINIKKNNSFNPTQYLEEVK EPTTVVATNIDIELWKKHYDFKIYSWNGTFEFRGSHGFFDTFVDHFMEIKKNSTG GLRQIAKLILNSLYGKFATNPDITGKHPTLKDNRVSLVMNEPETRDPVYTPMGVF ITAYARKKTISAAQDNYETFAYADTDSLHLIGPTTPPDSLWVDPVELGAWKHESS FTKSVYLRAKQYAEEIGGKLDVHIAGMPRNVAATLTLEDMLHGGTWNGKLIPVRV PGGTVLKDTTFTLKID |
| NO: 36 | MVRQSTIASPARGGVRRSHKKVPSFCADFETTTDEDDCRVWSWGIIQVGKLQNYV DGISLDGFMSHISERASHIYFHNLAVDGTFILDWLLKHGYRWTKENPGVKEFTSL ISRMGRYYSITVVFETGFRVEFRDSFKKLPMSVSAIAKAFNLHDQKLEIDYEKPR PIGYIPTEQEKRYQRNDVAIVAQALEVQFAEKMTKLTAGSDSLATYKKMTGKLFI RRFPILSPEIDTEIRKAYRGGFTYADPRYAKKLNGKGSVYDVNSLYPSVMRTALL PYGEPIYSEGAPRTNRPLYIASITFTAKLKPNHIPCINIKKNNSFNPTQYLEEVK EPTTVVATNIDIELWKKHYDFKIYSWNGTFEFRGSHGFFDTFVDHFMEIKKNSTG GLRQIAKLILNSLYGKFATNPDITGKHPTLKDNRVSLVMNEPETRDPVYTPMGVF ITAYARKKTISAAQDNYETFAYADTDSLHLIGPTTPPDSLWVDPVELGAWKHESS FTKSVYIRAKQYAEEIGGKLDVHIAGMPRNVAATLTLEDMLHGGTWNGKLIPVRV PGGTVLKDTTFTLKID |

## Claims

1. A DNA polymerase mutant comprising any one of the following mutations or any combination thereof relative to a wild-type polymerase having an amino acid sequence shown in SEQ ID NO: 1: T272S, T272Y, A273K, T371D, Y372F, L265A, I379K, L398T, M377Y, E378Q, T384K, L398S, E493K, E493Q, K491R, Q389R, S397T, L468I, G512Q, Y161L, M198A, L318N, Q313C, S319K, E162L, S24R, I67A, M114T, T210D, E233K, K491R, K116R, K315R, K316R, S319C, N317K, S319R, S319F, E493K, L265A, E378Q, L468I, L265A, N263Q, E493K, D516K, N151C, T384S, L265A, T384N, Q193C, I502L, D516A, Q193S, N151V, D516Y, D516G, D516N, N151L, Q193L, E509V, D516T, H518L, N151Q, D516S, N151C, T243S, T243V, D516K, N151C, T384S, T384N, Q193C, I502L, L265A, D516A, Q193S, N151V, D516Y, D516G, D516N, N151L, Q193L, E509V, D516T, H518L, E493K, N151Q, D516S, N151C, T243S, K491R, and T243V.

2. The DNA polymerase mutant of claim 1, comprising any one of the following mutations or any combination thereof relative to the wild-type polymerase, thereby increasing its extension rate with a modified nucleotide: T272S, T272Y, A273K, T371D, Y372F, L265A, I379K, L398T, M377Y, E378Q, T384K, L398S, E493K, E493Q, K491R, Q389R, S397T, L468I, and G512Q.

3. The DNA polymerase mutant of claim 1 or 2, comprising any one of the following mutations or any combination thereof relative to the wild-type polymerase, thereby improving its thermal stability at room temperature: Y161L, M198A, L318N, Q313C, S319K, E162L, S24R, I67A, M114T, T210D, and E233K.

4. The DNA polymerase mutant of any of claims 1-3, comprising any one of the following mutations or any combination thereof relative to the wild-type polymerase, thereby increasing its enzyme-template binding ability: K491R, K116R, K315R, K316R, S319C, N317K, S319R, S319F.

5. The DNA polymerase mutant of any of claims 1-4, comprising any one of the following mutations or any combination thereof relative to the wild-type polymerase, thereby being able to use a Mg²⁺ ion to catalyze a polymerase synthesis reaction: L265A and N263Q.

6. The DNA polymerase mutant of any of claims 1-5, comprising any one of the following mutations or any combination thereof relative to the wild-type polymerase, thereby increasing its extension rate and extension activity under the condition of high salt: E493K, D516K, N151C, T384S, L265A, Q193C, I502L, E509V, H518L, T243V.

7. The DNA polymerase mutant of any of claims 1-6, comprising any one of the following mutations or any combination thereof relative to the wild-type polymerase, thereby improving DNA polymerase extension processivity: D516K, N151C, T384S, T384N, Q193C, I502L, L265A, D516A, Q193S, N151V, D516Y, D516G, D516N, N151L, Q193L, E509V, D516T, H518L, E493K, N151Q, D516S, N151C, T243S, K491R, and T243V.

8. The DNA polymerase mutant of any of claims 1-7, comprising two or more of the following mutations relative to the wild-type polymerase: K116R, K315R, K316R, N317R, S319F, L265A, L265Y, E378Q, T384K, Q389R, K491R, E493Q, E493K, L468I, G512Q, D516R, T272S, T272Y, A273K, T371D, Y372F, I379K, L398T, M377Y, L398S, Y161L, M198A, L318N, Q313C, S268A, T272S, N263Q, H394I, F81I, F81V, and E30A.

9. The DNA polymerase mutant of any of claims 1-8, comprising any one of the following mutation combinations relative to the wild-type polymerase:
1) M198A+E378Q+N263Q+F460I+H518R;
2) M198A+Y372S+T384K+E493K+N263Q;
3) M198A+Y372S+T384K+K491R+G44Q;
4) L318N+M198A+T384K+E378Q+F81V;
5) E30A+L318N+E378Q+T384K+Q389R;
6) L265A+M114V+T384K+G512Q+T442K;
7) A273K+N317R+E378Q+K491R+I379K;
8) A273K+N317R+E378Q+E493K+I379K;
9) L265A+Q313C+E30A+H394I+E491M;
10) L265A+E378Q+T384K+K491R+E493K+F81I;
11) L265A+E378Q+T384K+K491R+E493K+F81V;
12) L265I+Q313C+E30A+H394I+K491M;
13) L265V+Q313C+E30A+H394I+E493V;
14) L265I+F219Y+K491R+K380R+I502L;
15) L265I+F81V+T384K+F219Y+I502L;
16) L265A+E378Q+T384K+K491R+E493K;
17) T272S+E378Q+F219Y+L318N+D516R;
18) G44Q+T272S+E378D+E493K+G512Q;
19) D34Q+T272Y+E378Q+M377Y+K491R;
20) T272Y+E378Q+M377Y+K491R+G512Q;
21) D34Q+Q313C+K491R+F81I+D516R;
22) G44Q+H394I+K491R+F81V+I502L;
23) T371D+E378Q+T384K+F81I+G512Q;
24) T371D+E378Q+T384K+F81V+D516R;
25) K116R+Y372F+L318N+H394I+Q313Y;
26) K116R+Y372F+L318N+H394I+Q313N;
27) S268N+Y161L+L398T+L265A+T384K;
28) L265A+E378Q+T384K+K491R+E493K+F81I+I502L;
29) L265A+E378Q+T384K+Q389R+K491R+E493Q+D516R;
30) T272Y+E378Q+M377Y+K491R+G512Q+F81I;
31) T272Y+E378Q+M377Y+K491R+G512Q+F81V;
32) T272Y+E378Q+M377Y+K491R+G512Q+F81I+I502L;
33) G44Q+T272S+E378Q+E493K+G512Q+D516R;
34) K116R+Y372F+L318N+H394I+Q313N+I502L; and
35) K116R+Y372F+L318N+H394I+Q313N+F81V.

10. The DNA polymerase mutant of any of claims 1-9, comprising
1) an amino acid sequence shown in any of SEQ ID NOs: 2-36; or
2) an amino acid sequence having at least 70% amino acid sequence identity to an amino acid sequence shown in any of SEQ ID NOs: 2-36 and having a DNA polymerase activity.

11. The DNA polymerase mutant of any of claims 1-10, wherein the mutant is fused with a SpyCatcher protein.

12. A method for sequencing a nucleic acid molecule, comprising using the DNA polymerase mutant of any of claims 1-11 in a sequencing process.

13. The method of claim 12, wherein the method uses sequencing-by-synthesis.

14. The method of claim 11 or 12, wherein the sequencing is single-molecule sequencing.

15. The method of any of claims 12-14, wherein the sequencing is single-molecule sequencing using a nanopore.

16. The method of any of claims 12-15, wherein the nanopore is fused with a SpyTag.

17. The method of any of claims 12-16, wherein the DNA polymerase mutant is conjugated to the nanopore.

18. The method of any of claims 12-17, wherein the method performs DNA synthesis using four nucleotide monomers bearing different modifying groups, so as to sequentially identify different nucleotide residues incorporated into a newly synthesized DNA single strand; preferably, the modifying groups are attached to the phosphate groups of the nucleotide monomers.

19. The method of any of claims 12-18, wherein the sequencing-by-synthesis is performed under conditions containing 60-200 mM KCl.

20. The method of any of claims 12-19, wherein the sequencing-by-synthesis is performed under conditions containing Mg²⁺; or the sequencing-by-synthesis is performed under conditions containing Mg²⁺ and free of Mn²⁺.

21. A kit or device for replication, amplification, or sequencing of a nucleic acid molecule, comprising the DNA polymerase mutant of any of claims 1-11.

22. The kit or device of claim 21, wherein the sequencing is performed by sequencing-by-synthesis.

23. The kit or device of claim 21 or 22, wherein the sequencing is single-molecule sequencing.

24. The kit or device of any of claims 21-23, wherein the sequencing is single-molecule sequencing using a nanopore.

25. The kit or device of any of claims 21-24, further comprising four types of nucleotide monomers with different modifying groups; preferably, the modifying groups are attached to phosphate groups of the nucleotide monomers.

26. The kit or device of any of claims 21-25, wherein the DNA polymerase mutant is conjugated to the nanopore.
